# EUROPÄISCHE PATENTSCHRIFT

(11) **EP 4 333 752 B1**
(45) Veröffentlichungstag und Bekanntmachung des Hinweises auf die Patenterteilung: **25.12.2024**
(21) Anmeldenummer: 23701003.8
(22) Anmeldetag: 16.01.2023
(51) Int. Cl.: A61B 18/14

(54) **CAIMAN RING FORCEPS - MANUELLE HF AKTIVIERUNG**
CAIMAN RING FORCEPS - MANUAL HF ACTIVATION
ACTIVATION HF MANUELLE DE FORCEPS CROCODILE À ANNEAUX

(30) Priorität: 17.01.2022 DE 102022100924
(43) Veröffentlichungstag der Anmeldung: 13.03.2024
(73) Patentinhaber: Aesculap AG, 78532 Tuttlingen (DE)
(72) Erfinder: HAFNER, Nikolaus, 78532 Tuttlingen (DE); BAUER, Markus, 72184 Eutingen-Rohrdorf (DE); GIORDANO, Nicola, 78056 Villingen-Schwenningen (DE)
(74) Vertreter: Winter, Brandl - Partnerschaft mbB
(86) Internationale Anmeldenummer: PCT/EP2023/050842
(87) Internationale Veröffentlichungsnummer: WO 2023/135288

(56) Entgegenhaltungen:
- EP-A1- 3 488 805
- WO-A1-2014/158458
- WO-A1-2019/224634

## Beschreibung

### Technisches Gebiet

Die vorliegende Offenbarung betrifft ein (medizinisches) bipolares HF-Instrument vorzugsweise der Scheren- oder Zangenbauart mit einer ersten und einer zweiten (in einem Scharnier sich kreuzenden) Instrumentenbranchen, die an einem distalen Instrumenten-Endabschnitt ein Maulteil beispielsweise zum Fassen von Gewebe und an einem proximalen Instrumenten-Endabschnitt Halte-/Betätigungsgeometrien (Griffe, Fingerringe, etc.) ausbilden oder aufweisen, welche sämtlich dazu vorgesehen und ausgebildet sind, sich beim Öffnen voneinander weg und beim Schließen aufeinander zu zubewegen zum manuellen Schneiden und (HF-) Versiegeln von Gewebe insbesondere in der offenen Chirurgie.

### Hintergrund der Offenbarung

Im Allgemeinen sind chirurgische Versiegelungs- und Schneideinstrumente der vorstehenden Gattung bekannt, welche einen hochfrequenten (HF) Strom für die Versiegelung mittels bipolarer Technik und eine mechanische Klinge für den Schnitt von (biologischem) Gewebe verwenden. Hierbei ist eine automatische Daueraktivierung beim Zusammendrücken der Branchen vorgesehen. Häufig ist eine Klingenverriegelung im offenen Zustand der Branchen als Sicherheitsvorkehrung in das Instrument integriert. Ein des Weiteren vorgesehener HF-Taster vermittelt in der Regel ein taktiles Feedback in zwei Stufen (2-Klick-Taster). Bei einem ersten Klick wird einem Anwender ein Hinweis gegeben, dass ein definierter Schließ-Zustand erreicht ist und bei einem zweiten Klick wird der HF-Strom aktiviert. Mit diesem chirurgischen Instrument aus dem Stand der Technik ist eine schnelle Arbeitsweise möglich, insbesondere für erfahrene Anwender. Das chirurgische Instrument weist klar getrennte Funktionen zum Schneiden und Versiegeln auf sowie geringe Herstellungskosten.

Alternativ gibt es ein chirurgisches Instrument, welches Ultraschall für die Versiegelung und den Schnitt verwendet, wobei die Versiegelung und der Schnitt manuell dauer-aktivierbar sind. Ferner bietet dieses chirurgische Instrument die Wahl zwischen einem MIN- oder einem MAX-Modus an dem Instrument. Es ist von Vorteil, dass beim Schließen keine ungewollte Aktivierung möglich ist, insbesondere da der Anwender freie Auswahl hat und dadurch mehr Kontrolle und Sicherheit gegeben ist.

Die bekannten chirurgischen Instrumente haben jedoch den Nachteil, dass ein permanentes Halten des HF-Tasters notwendig ist und dies für einen Anwender auf Dauer unergonomisch und ermüdend sein kann, insbesondere, da mehrere Aktivierungen pro Operation denkbar sind. Ein weiterer Nachteil kann die Variation des aufgebrachten Drucks sein. Der Anwender kann nämlich durch seine Handkraft den Betätigungsdruck auf das Instrument/dessen Branchen variieren und somit ggf. das Versiegelungsergebnis negativ beeinflussen. Eine versehentliche HF-Aktivierung bei zu schnellem Zusammendrücken sowie eine bevorzugte Ausrichtung bei der Handhabung aufgrund der asymmetrischen Anordnung des Klingenbetätigungselements sind weitere Nachteile.

### Stand der Technik

Die US 10 188 450 B2 beschreibt eine Zange mit einem ersten und einem zweiten Schaft (Branche), der jeweils ein Backenelement aufweisen, das an einem distalen Ende davon angeordnet ist. Mindestens ein Backenelement ist von einer offenen in eine geschlossene Position bewegbar, um Gewebe dazwischen zu greifen. Mindestens ein Backenelement ist zum hin- und her Bewegen einer Klinge konfiguriert. Eine Auslöseranordnung umfasst einen Auslöser und mindestens ein Gestänge, das mit dem Auslöser und der Klinge verbunden ist, so dass eine Drehung des Auslösers die Klinge zwischen der eingefahrenen und der ausgefahrenen Position verschiebt. Ein Eingriffselement, das zwischen einer verriegelten Position und einer unverriegelten Position beweglich ist, wird ebenfalls bereitgestellt. Das Eingriffselement ist so konfiguriert, dass es in der verriegelten Position mit dem/den Gestänge(n) in Eingriff steht, um eine Verschiebung der Klinge aus der eingefahrenen in die ausgefahrene Position zu verhindern.

Die US 10 660 694 B2 beschreibt eine Schalteranordnung für ein elektrochirurgisches Instrument mit einem Schaltergehäuse, einem Schalter, einem ersten Vorspannelement und einem zusätzlichen Vorspannelement. Der Schalter ist innerhalb des Schaltergehäuses angeordnet und zwischen einer aktivierten Position zum Einleiten der Abgabe elektrochirurgischer Energie und einer deaktivierten Position zum Beenden der Abgabe elektrochirurgischer Energie beweglich angeordnet. Das erste Vorspannelement ist selektiv neben dem Schalter positionierbar und steht damit in Verbindung. Das erste Vorspannelement umfasst eine erste Dicke, die einen ersten Widerstand bereitstellt, um einer Bewegung des Schalters zwischen der aktivierten und der deaktivierten Position zu widerstehen, wenn er in dem Schaltergehäuse positioniert ist. Das zusätzliche Vorspannelement ist selektiv mit dem ersten Vorspannelement austauschbar. Das zusätzliche Vorspannelement weist eine unterschiedliche Dicke auf, die einen unterschiedlichen Widerstand bereitstellt, um einer Bewegung des Schalters zwischen der aktivierten und der deaktivierten Position zu widerstehen, wenn er in dem Schaltergehäuse positioniert ist.

Aus der EP 2 671 528 B1 und der EP 2 436 330 B1 ist ein bipolares elektrochirurgisches Instrument bekannt, mit einem ersten und einem zweiten Schaft (Branche), der jeweils ein Backenelement aufweist, das sich von seinem distalen Ende erstreckt. Jedes Backenelement ist angepasst, um eine Verbindung mit einer Quelle elektrochirurgischer Energie herzustellen, so dass die Backenelemente in der Lage sind, Energie selektiv durch das dazwischen gehaltene Gewebe zu leiten. Ein Messerkanal ist so konfiguriert, dass er einen Schneidmechanismus darin hin- und her bewegt. Ein Aktuator schiebt selektiv den Schneidemechanismus vor. Ein Schalter ist an der ersten Welle angeordnet und konfiguriert, um zwischen einer ersten Position und mindestens einer nachfolgenden Position bei einem Vorspanneingriff mit einer mechanischen Schnittstelle, die an der zweiten Welle angeordnet ist, niedergedrückt zu werden. Die erste Position des Schalters leitet Informationen an den Benutzer weiter, die einem gewünschten Druck auf das Gewebe entsprechen, und die mindestens eine nachfolgende Position ist konfiguriert, um die Quelle elektrochirurgischer Energie zu aktivieren, um den Backenelementen elektrochirurgische Energie zuzuführen.

Die US 7 253 667 B2, US 9 498 279 B2, US 2019 0356 952 A1, WO 2019 224 634 A1, EP 1 609 430 B1, US 2019 0357 969 A1, WO 2019 224 636 A3, US 2019 0357 967 A1 und WO 2019 224 637 A1 beschreiben ebenfalls gattungsgemäße elektrochirurgische Instrumente nach dem einleitenden Teil des Anspruchs 1. Dabei offenbart die oben genannte WO 2019 224 634 A1 beidseitige Bedienmöglichkeiten.

### Kurzbeschreibung der Offenbarung

Der vorliegenden Offenbarung liegt die Aufgabe zugrunde, ein bipolares HF-Instrument bereitzustellen, welches eine vereinfachte Bedienung, insbesondere über einen längeren Zeitraum ermöglicht, und ferner die Ausrichtung des Instruments zur Verwendung irrelevant ist. Ferner ist es insbesondere ein Ziel der vorliegenden Offenbarung, die vorstehenden Nachteile aus dem Stand der Technik zu beseitigen oder zumindest zu verbessern.

Diese Aufgabe wird gelöst durch ein bipolares HF-Instrument mit den Merkmalen des Anspruchs 1.

Demnach hat das bipolare HF- (Hand-Instrument ein Maulteil (zum Fassen! Festhalten! Greifen von Gewebe), sowie eine erste Instrumentenbranche und eine zweite Instrumentenbranche (zum manuellen Betätigen des Maulteils), welche (sämtlich) dazu vorgesehen und ausgebildet sind, sich beim Öffnen voneinander weg und beim Schließen aufeinander zu zubewegen. Darüber hinaus ist ein Klingenbetätigungselement/ Klingenbetätigungshebel oder (Schiebe-)Taste zum Aktivieren einer in dem Maulteil gelagerten mechanischen Klinge zum Schneiden von Gewebe vorgesehen, wobei die mechanische Klinge dazu vorgesehen und ausgebildet ist, um mittels des Klingenbetätigungselements von einer ersten Position (Park-/Nicht-Schneideposition) in eine zweite Position (Schneideposition) bewegbar zu sein. Des Weiteren ist ein HF-Aktivierungselement oder Aktivierungstaste/-knopf/ -schalter zum Aktivieren/Freischalten einer HF-Stromzufuhr insbesondere zum Versiegeln (Koagulieren) von Gewebe und ggf. eine, vorzugsweise separat dazu ausgebildete, sowie auf die Branchen einwirkende Einrastvorrichtung vorgesehen, um (wahlweise) einen eingerasteten Branchenzustand bei geschlossenen Instrumentenbranchen zu erreichen.

Der Grundgedanke der vorliegenden Offenbarung besteht nunmehr darin, dass das HF-Aktivierungselement und vorzugsweise das Klingenbetätigungselement derart vorgesehen und ausgebildet sind, um jeweils von zwei, vorzugsweise jeweils sich gegenüberliegenden (bzw. voneinander abgewandten) Seiten des HF-Instruments in weiter vorzugsweise gleicher oder gleichartiger Weise bedienbar zu sein. Dies hat den Vorteil, dass die mechanische Klinge, egal wie herum bzw. in welcher Hand das Instrument gehalten wird, immer gleichartig betätigt werden kann. Demnach gibt es keine bevorzugte Ausrichtung bzw. Haltung des Instruments. Dies gilt ebenso für die Handhabung als Rechts- oder Linkshänder. In anderen Worten, ist es irrelevant, wie ein Anwender das bipolare HF-Instrument in die Hand nimmt, da die Bedienung durch die beidseitige Ausbildung des Klingenbetätigungselements als auch des HF-Aktivierungselements immer in der gleichen Weise funktioniert.

In anderen Worten handelt es sich beim vorliegenden Gegenstand gemäß der Offenbarung um ein chirurgisches (Hand-Instrument der Scheren- oder Zangenbauart mit bipolarer HF-Technologie und mechanischer (Gewebeschneid-)Klinge. Durch das Öffnen und Schließen der Instrumentenbranchen wird das Maulteil ebenfalls geöffnet und geschlossen. Im geschlossenen Zustand kann Gewebe gefasst und mittels HF (hochfrequenter Energiezufuhr) über im Maulteil angeordneter Elektroden versiegelt werden. Mit dem Maulteil sind zudem Präparation und Manipulation an dem Gewebe möglich. Im geschlossenen Zustand des Maulteils kann das Gewebe mittels der im Maulteil gelagerten Klinge ebenfalls geschnitten werden. Die Betätigungseinrichtungen zur separaten und individuellen manuellen Betätigung der HF-Stromzufuhr und der Klinge sind derart ausgebildet und angeordnet, dass sich auf zwei jeweils gegenüberliegenden bzw. voneinander abgewandten Seiten des Instruments im Wesentlichen die gleiche Betätigungsgeometrie ergibt mit im Wesentlichen der gleichen Funktion/Wirkung auf die HF-Stromzufuhr und die Klinge. Hierfür wird ausdrücklich darauf hingewiesen, dass die vorstehend beschriebene Spiegelung der Betätigungsgeometrie nicht notwendiger Weise nur zweidimensional, sondern auch dreidimensional erfolgen kann. In anderen Worten ausgedrückt können beide Betätigungseinrichtungen zur Betätigung von HF-Strom und Klinge beispielsweise links und rechts des Instruments im Wesentlichen gleich (spiegelbildlich) angeordnet sein (zweidimensionale Duplikation) oder nur die eine Betätigungseinrichtung (ggf. Klingenbetätigung) links und rechts des Instruments und die andere Betätigungseinrichtung (ggf. HF-Stromzufuhr) oben und unten des Instruments (jeweils spiegelbildlich) angeordnet sein (dreidimensionale Duplikation).

Die mechanische Klinge ist mittels des Klingenbetätigungselements von einer ersten Position in eine zweite Position beweglich. Das Klingenbetätigungselement wird dabei linear von dessen distaler Ruhe-Parkposition nach proximal bewegt. Die mechanische Klinge bewegt sich entsprechend linear von dessen proximaler Ruheposition nach distal und kann so im Maulteil gefasstes Gewebe schneiden.

Weiterhin hat das Instrument bevorzugt eine fest eingebaute Einrastvorrichtung in Form einer Durchlaufsperre. Mit Hilfe der Raste können die Instrumentenbranchen in einem definierten Zustand (Relativposition) arretiert werden. Zum Einrasten werden die Instrumentenbranchen geschlossen, bis der eingerastete Zustand erreicht wird. Zum Ausrasten werden die Branchen wieder etwas zusammengedrückt, bis sich die Raste löst. Die Gestaltung der Einrastvorrichtung/ Raste/ Durchlaufsperre ist aus dem Stand der Technik hinlänglich bekannt und muss daher nicht weiter beschrieben werden. Sie hat den wesentlichen Vorteil, dass mit derer Hilfe ein definierter Schließzustand erzeugbar ist, bei dem Schwankungen der Schließkraft nur in geringem Maße zu erwarten sind. Dies wiederum hat den Vorteil, dass die Versiegelungsqualität erhöht bzw. eine konstante Performance sichergestellt ist. Zudem ermöglich die Einrastvorrichtung dem Anwender, Gewebe sicher zu halten, ohne dass dieser eine HF-Aktivierung auslöst. Ein weiterer Vorteil ist, dass der Anwender bei geschlossener Raste seine Hand entspannen kann. Es ist kein permanentes Zusammendrücken des Instruments notwendig. Damit ist diese Offenbarung besonders ergonomisch.

Weitere Aspekte der vorliegenden Offenbarung sind nachfolgend gemäß den Unteransprüchen beschrieben.

Es ist von Vorteil, wenn die Einrastvorrichtung aus einem männlichen Teil und einem weiblichen Teil besteht. In anderen Worten kann im geschlossenen und eingerasteten Zustand HF aktiviert und geschnitten werden. In der Regel wird zuerst HF aktiviert und eine Versiegelung durchgeführt. Hierzu wird das HF-Aktivierungselement nach proximal bewegt. Es ist bevorzugt eine Tipp-Aktivierung vorgesehen, sodass der Anwender nur einmalig den Taster/Knopf betätigen muss und diesen nicht permanent halten muss. Ein HF-Generator übernimmt mit seinem integrierten Algorithmus die Logik und schaltet nach einer erfolgreichen Versiegelung selbständig aus. Der Anwender hat jedoch immer die Möglichkeit, durch eine erneute Tipp-Aktivierung des HF-Aktivierungstasters/-knopfs den Prozess der Versiegelung zu stoppen. In einem zweiten Schritt kann geschnitten werden. Hierzu wird das Klingenbetätigungselement nach proximal gezogen. Sowohl das HF-Aktivierungselement als auch das Klingenbetätigungselement werden automatisch zurückgestellt, vorzugsweise durch Federn.

Es ist bevorzugt, wenn die Funktionen der Versiegelung und/oder des Schneidens unabhängig voneinander benutzbar sind. In anderen Worten hat der Anwender die Freiheit, die Funktionen Versiegeln und/ oder Schneiden unabhängig voneinander zu benutzen.

Es ist bevorzugt, wenn in der ersten Instrumentenbranche ein erstes Zahnstangen-Zahnrad-Getriebe vorgesehen und angeordnet ist, bei welchem eine Input-Zahnstange mit dem Klingenbetätigungselement und eine Output-Zahnstange mit der mechanischen Klinge verbunden ist, wobei das Klingenbetätigungselement linear von einer distalen Ruheposition nach proximal bewegbar ist. In anderen Worten ist die dafür benötigte Umlenkmechanik in der ersten Instrumentenbranche ein Zahnstangen-Zahnrad-Getriebe, bei dem die Input-Zahnstange an dem Klingenbetätigungselement und die Output-Zahnstange an der mechanischen Klinge befestigt ist.

Es ist von Vorteil, wenn die Input-Zahnstange und das damit verbundene Klingenbetätigungselement annähernd auf der Symmetrie-Ebene zwischen der ersten Instrumentenbranche und der zweiten Instrumentenbranche angeordnet sind.

In anderen Worten stellt die Funktionsweise mit dem Zahnstangen-Zahnrad-Getriebe einen wesentlichen Vorteil für die symmetrische Nutzung des Instruments dar. Die Anordnung der Input-Stange und des damit verbundenen Klingenbetätigungselements annähernd auf der Symmetrie-Ebene zwischen erster und zweiter Instrumentenbranche bringt den großen Vorteil, dass die Klinge, egal wie herum das Instrument gehalten wird, immer gleich betätigt werden kann. Es gibt keine bevorzugte Ausrichtung bzw. Haltung des Instruments in dieser Hinsicht. Dies gilt ebenso für die Handhabung als Rechts- oder Linkshänder.

Es ist bevorzugt, wenn das bipolare HF-Instrument zudem eine Klingenverriegelung hat, welche derart konfiguriert ist, um in einem offenen Zustand die Betätigung der mechanischen Klinge zu blockieren und dass in einem definierten Schließzustand die mechanische Klinge beweglich ist. Hierbei sind zwei verschiedene Sicherheitskonzepte vorgesehen, welche in das bipolare HF-Instrument implementierbar sind.

In einem ersten Sicherheitskonzept ist vorgesehen, dass eine Klingenbetätigung möglich ist, sobald die Maulteile ausreichend geschlossen sind, beispielsweise, wenn sie sich berühren. Eine HF-Aktivierung/-Betätigung ist prinzipiell immer möglich. Das heißt, es gibt keine Verriegelung des HF-Aktivierungselements. Hierbei ist alternativ vorgesehen, dass HF aktiviert werden kann, wenn das Instrument nicht eingerastet ist. Dies ermöglich dem Anwender mehr Anwendungsmöglichkeiten, birgt aber auch das Risiko, dass es zu einer HF-Aktivierung mit nicht ausreichender Schließkraft kommt. Hierbei ermöglicht die feste Einrastvorrichtung einen definierten Schließzustand.

In einem zweiten Sicherheitskonzept ist sowohl die Klingenbetätigung als auch die HF-Betätigung nur möglich, wenn sich das Instrument in der eingerasteten Position der fest eingebauten Einrastvorrichtung befindet. Dies stellt sicher, dass sowohl ein Schnitt als auch eine HF-Versiegelung nur in einem klar definierten Schließzustand möglich sind und gewährleistet maximale Sicherheit. Diese Option schränkt den Anwender jedoch in seinen Möglichkeiten ein. Besonders erfahrene Anwender könnten dieses Sicherheitskonzept als starke Limitierung wahrnehmen. Daher ist es bevorzugt, wenn das erste Sicherheitskonzept für erfahrene Anwender und das zweite Sicherheitskonzept für weniger erfahren Anwender verwendet wird.

Bevorzugter Weise ist das HF-Aktivierungselement derart ausgebildet und vorgesehen, um bei Durchführung einer Versiegelung nach proximal bewegbar oder verschwenkbar oder verschiebbar zu sein.

Es ist vorteilhaft, wenn das HF-Aktivierungselement derart ausgebildet und vorgesehen ist, um nach einer ersten Tipp-Aktivierung die HF-Stromzufuhr zu aktivieren und automatisch, vorzugsweise mittels eines integrierten Algorithmus, nach erfolgreicher Versiegelung auszuschalten oder um nach einer zweiten Tipp-Aktivierung des HF-Aktivierungselements vor erfolgreicher Versiegelung, die HF-Stromzufuhr manuell zu stoppen.

Erfindungsgemäß hat das HF-Aktivierungselement einen Innenteil, der wippend, schiebbar oder verschwenkbar auf einer Drehachse des HF-Aktivierungselements in der zweiten Instrumentenbranche orthogonal zu der proximal-distal-Richtung gelagert ist, wobei die in Richtung proximal des Instruments weisende Seite des Innenteils ein Kontaktierungselement mit einer Aktivierungsfläche aufweist, welche einen ersten HF-Aktivierungstaster bei Betätigung des HF-Aktivierungselements kontaktiert und die HF-Stromzufuhr aktiviert.

In anderen Worten verfolgt die Gestaltung und Positionierung des HF-Betätigungstasters ebenfalls das Ziel, die Bedienung des Instruments vollsymmetrisch zu ermöglichen, bzw. die Notwendigkeit einer bevorzugten Ausrichtung des Instruments zu vermeiden.

Gemäß einer ersten bevorzugten Ausführungsform ist das HF-Aktivierungselement um eine Achse herum wippend in der zweiten Branche gelagert und kann von beiden Seiten des Instruments gleich gut erreicht werden. Ferner bringt die Positionierung des HF-Aktivierungselements in nur einer Instrumentenbranche den Vorteil, dass sich die Verkabelung der HF-Aktivierung ebenfalls nur in einer Instrumentenbranche befindet. Dies reduziert den Aufwand und die Kosten gegenüber einem Konzept mit Betätigungstastern/HF-Aktivierungsknöpfen in beiden Instrumentenbranchen signifikant.

Erfindungsgemäß hat der Innenteil des HF-Aktivierungselements einen ersten Flügel und einen zweiten Flügel, wobei der erste Flügel seitlich an der zweiten Instrumentenbranche hervorsteht und der zweite Flügel dem ersten Flügel gegenüberliegend auf der anderen Seite der zweiten Instrumentenbranche hervorsteht.

In anderen Worten besteht das HF-Aktivierungselement aus mehreren Teilen, einem Innenteil und einem ersten sowie einem zweiten Flügel. Alternativ ist unter anderen Montagebedingungen auch eine einteilige Ausführung denkbar.

Alternativ ist es denkbar, das HF-Aktivierungselement als Schieber zu gestalten, der in proximaler und distaler Richtung linear bewegbar ist. Hierzu ist es vorteilhaft, wenn das HF-Aktivierungselement in beispielsweise einem Schlitz in einem Gehäuse der zweiten Instrumentenbranche beweglich geführt ist. Dies hat den Vorteil, dass genau ein elektronischer HF-Aktivierungstaster notwendig ist.

Die Aktivierungsfläche des HF-Aktivierungselements gemäß der vorstehenden Ausführungsform ist dazu vorgesehen, auf den ersten (elektronischen) HF-Aktivierungstaster zu drücken.

Erfindungsgemäß ist das Kontaktierungselement nockenförmig in Richtung hin zu dem ersten HF-Aktivierungstaster vorgesehen und ausgebildet.

Bei einem alternativen nicht zur Erfindung gehörenden Beispiel ist ein zweites Zahnstangen-Zahnrad-Getriebe derart in Richtung distal zu dem ersten HF-Aktivierungstaster platziert, um diesen bei Betätigung des HF-Aktivierungselements mit der Aktivierungsfläche zu kontaktieren.

In anderen Worten ist eine alternative Ausführungsform mit genau einem elektronischen HF-Aktivierungstaster möglich. Hierzu weist der Innenteil des HF-Aktivierungselements eine Art Nocke oder ähnliches auf, die bei der Rotation/Verschwenkung um die Drehachse des HF-Aktivierungselements in Eingriff mit dem ersten HF-Aktivierungstaster kommt. Hierbei ist eine zusätzliche Rückstellfeder vorgesehen und ggf. notwendig.

Eine alternative nicht zur Erfindung gehörende Ausführungsform bei Verwendung (genau) eines ersten HF-Aktivierungstasters ist mit einem Zahnstangen-Zahnrad-Getriebe vorgesehen. Dabei ist das Innenteil des HF-Aktivierungselements ebenfalls drehbar gelagert und mit einem Zahnrad verbunden. Auf zwei Seiten des Zahnrades sind entsprechend zwei Zahnstangen platziert, die jeweils ihre Bewegung auf ein Kontaktierungselement/ Aktivierungselement/ eine Druckplatte übertragen können. Das Kontaktierungselement betätigt dann den ersten HF-Aktivierungstaster. Je nach Ausführung des HF-Aktivierungstasters ist das Kontaktierungselement überflüssig und die Zahnstangen können direkt auf den HF-Aktivierungstaster drücken. Um eine zuverlässige Funktion zu ermöglichen, ist es von Vorteil, wenn die Zahnstangen mit Rückstellfedern versehen sind.

Es ist vorteilhaft, wenn ein zweiter HF-Aktivierungstaster neben dem ersten HF-Aktivierungstaster parallel zu dem Innenteil des HF-Aktivierungselements vorgesehen und angeordnet ist. In anderen Worten übt die Aktivierungsfläche des Kontaktierungselements durch das Verdrehen/ Verschwenken/ Verschieben des HF-Aktivierungselements eine Kraft auf einen der beiden HF-Aktivierungstaster aus und löst damit eine Aktivierung des HF-Prozesses aus. Durch erneutes Verdrehen/ Verschwenken/ Verschieben des HF-Aktivierungselements kann der HF-Prozess bei Bedarf auch manuell gestoppt werden. Neben der vollsymmetrischen Gestaltung hat die vorstehende Ausführungsform den Vorteil, dass das HF-Aktivierungselement sowohl durch Ziehen als auch durch Drücken betätigbar ist/betätigt werden kann. Dies eröffnet dem Anwender mehrere ergonomische Bedien-Optionen.

Es ist bevorzugt, wenn eine Rückstellung in eine nicht betätigte Ruheposition über eine integrierte Feder in den HF-Aktivierungstastern vorgesehen ist. Alternativ dazu kann eine zusätzliche Rückstellfeder in Form einer Blech-/Blattfeder, einer Torsionsfeder oder ähnlichem erfolgen.

Es ist demnach vorteilhaft, wenn das Innenteil des HF-Aktivierungselements mit einem Federelement, beispielsweise einer Druckfeder, unterbaut ist. Dies hat den Vorteil, dass das Federelement bewirkt, dass das gesamte HF-Aktivierungselement ein Stück weit in Axialrichtung seiner Drehachse herausragt. Dies hat zur Folge, dass die Aktivierungsfläche, die auf den ersten und/ oder zweiten HF-Aktivierungstaster drücken würde, außer Eingriff gebracht ist und beim Betätigen des HF-Aktivierungselements in dieser Position also keine HF-Energiezufuhr aktivierbar ist. In einem geschlossenen Zustand wird das HF-Aktivierungselement in der zweiten Instrumentenbranche gedrückt. In dieser Position wird die Aktvierungsfläche durch Schließen der ersten und zweiten Instrumentenbranchen und das dadurch bedingte Zusammendrücken der Druckfeder in die zweite Instrumentenbranche wieder in Eingriff gebracht mit dem ersten und/oder zweiten HF-Aktivierungstaster. Hierbei ist es von Vorteil, wenn das HF-Aktivierungselement eine separate Rückstellfeder besitzt, die diesen permanent in eine Ruhestellung drängt, bei der die Flügel des HF-Aktivierungselements senkrecht zur Hauptebene des Instruments stehen. Als Rückstellfeder kann eine Blattfeder, Zugfeder, Torsionsfeder, oder ähnliches genommen werden.

Es ist von Vorteil, wenn für den ersten HF-Aktivierungstaster und/ oder den zweiten HF-Aktivierungstaster ein handelsüblicher elektronischer Taster verwendbar ist.

Es ist bevorzugt, wenn der erste HF-Aktivierungstaster und/oder der zweite HF-Aktivierungstaster in proximaler Richtung weg von dem Innenteil des HF-Aktivierungselements diesem jedoch zugewandt auf einer Platine in der zweiten Instrumentenbranche angeordnet ist/ sind. In anderen Worten ist es bevorzugt, dass sich der erste HF-Aktivierungstaster und/ oder zweite HF-Aktivierungstaster auf einer Platine befinden, auf welcher sie in einer ODER-Logik miteinander verschaltet sind. So ist gewährleistet, dass egal welcher der HF-Aktivierungstaster gedrückt wird, eine Aktion erfolgt.

Alternativ ist es bevorzugt, wenn das HF-Aktivierungselement hebelartig ausgeführt ist. Hierbei ist es vorgesehen, dass die Position eines derartigen Hebels umschaltbar ist, sobald das bipolare HF-Instrument gedreht wird, sodass auch eine vollsymmetrische Betätigung des Instruments möglich ist. Daher befindet sich der Hebel auf beiden Seiten des Instruments bzw. der zweiten Instrumentenbranche. Diesbezüglich ist es vorteilhaft, wenn in der Instrumentenbranche bzw. in dem Gehäuse der Instrumentenbranche ein erster und ein zweiter HF-Aktivierungstaster vorgesehen! platziert sind, welche sich derart gegenüberstehen, dass, wenn sich der Hebel in einer ersten Position befindet, der erste HF-Aktivierungstaster bei Betätigung gedrückt wird, und wenn sich der Hebel in Position 2 befindet, der zweite HF-Aktivierungstaster bei Betätigung gedrückt wird. Eine Umschaltbarkeit von Position 1 zu Position 2 ist bevorzugter Weise durch eine Schnappmechanik zu realisieren, damit der Hebel spürbar in den definierten Positionen einrastbar ausgebildet ist.

Es ist bevorzugt, wenn gemäß dem zweiten Sicherheitskonzept der Einbau einer HF-Aktivierungselement-Verriegelung vorgesehen ist. In anderen Worten wird dabei durch ein Verriegelungselement die Betätigung des HF-Aktivierungselements im offenen Zustand verhindert. In einem geschlossenen Zustand wird die Betätigung ermöglicht.

Es ist von Vorteil, wenn das Verriegelungselement einen Stößel hat, der aus einer Handgriffschale der zweiten Instrumentenbranche herausragt und mit einer gegenüberliegenden Handgriffschale der zweiten Instrumentenbranche in Kontakt bringbar ist, wenn das Instrument geschlossen wird. Neben dem Stößel ist ein Verriegelungselement, beispielsweise ein Stift, vorgesehen. Der Stift ist dazu vorgesehen und ausgebildet, um in Interaktion mit einer Bohrung im Innenteil des HF-Aktivierungselements zu stehen. In einem offenen Zustand befindet sich der Stift in der Bohrung und ein Verdrehen/ Betätigen des HF-Aktivierungselements ist nicht möglich. In einem geschlossenen Zustand wird durch den Stößel der Stift aus der Bohrung geschoben und das Verdrehen/ Betätigen des HF-Aktivierungselements ist möglich. Hierbei ist es bevorzugt, wenn das Verriegelungselement auf zwei Seiten mit Führungselementen ausgestattet/ ausgebildet ist, um ein Verdrehen des Verriegelungselements zu verhindern.

Es ist bevorzugt, wenn das Klingenbetätigungselement eine zum HF-Aktivierungselement unterschiedliche Form aufweist. Vorzugsweise ist das Klingenbetätigungselement rund ausgebildet und das HF-Aktivierungselement weist eine andere z.B. eine rechteckige Form auf. Ferner ist es zusätzlich oder alternativ bevorzugt, wenn das HF-Aktivierungselement eine andere Farbe, vorzugsweise blau, hat als das restliche Instrument, insbesondere da blau in der Chirurgie üblicherweise mit einer HF-Aktivierung assoziiert wird. Darüber hinaus ist es vorteilhaft, wenn das HF-Aktivierungselement zusätzlich oder alternativ mit einem ausreichenden Abstand zu dem Klingenbetätigungselement vorgesehen ist. Diese Unterschiede haben den Vorteil, dass eine Verwechslungsgefahr zwischen dem Klingenbetätigungselement und dem HF-Aktivierungselement so weit wie möglich reduzierbar ist.

Es ist bevorzugt, wenn der Abstand des Klingenbetätigungselements zu einem Griff der ersten Instrumentenbranche 76 ± 20 mm beträgt und der Abstand des HF-Aktivierungselements zu dem Griff der ersten Instrumentenbranche 42 ± 20 mm beträgt.

Es ist von Vorteil, wenn das HF-Aktivierungselement gegen eindringende Flüssigkeiten, insbesondere Blut, Wasser, Kochsalzlösung etc. abgedichtet ist. Es ist bevorzugt, wenn die erste und die zweite Handgriffschalen der ersten und/ oder zweiten Instrumentenbranche umlaufend mit einer Dichtung versehen ist. Alternativ oder zusätzlich ist es vorgesehen, wenn nur im Bereich des HF-Aktivierungselements eine Dichtung vorhanden ist. In anderen Worten ist aufgrund der Anwendung des Instruments in der offenen Chirurgie ein weiterer wichtiger Aspekt für das HF-Aktivierungselement die Abdichtung gegen eindringende Flüssigkeiten. Es muss daher sichergestellt werden, dass es beim Eindringen von Flüssigkeit nicht zu einer ungewollten HF-Aktivierung kommt. Hierzu ist vorgesehen das HF-Aktivierungselement entsprechend abzudichten. Hierbei ist eine Abdichtung der Handgriffschalen mit Dichtungen, beispielsweise TPE, bevorzugt. Die Dichtungen können an der gesamten Kontur der Handgriffschalen umlaufend sein, und/ oder nur im Bereich des HF-Aktivierungselements vorhanden sein. Die Dichtung kann in einer Handgriffschale mit angespritzt sein oder ein separates Teil sein, das bei der Montage mit eingelegt wird. Durch Verpressen oder Verschrauben mit der zweiten Handgriffschale wird die Dichtwirkung erreicht.

Alternativ oder zusätzlich ist es bevorzugt, wenn der verwendete erste und/ oder zweite HF-Aktivierungstaster bereits ab Werk dicht ausgebildet ist. In anderen Worten ist die Verwendung von abgedichteten HF-Aktivierungstastern, die ab Werk schon dicht sind, vorgesehen. Hierbei muss zusätzlich die Platine abgedichtet werden, auf der die HF-Aktivierungstaster aufgebracht/ aufgelötet sind, damit es nicht an den Anschlüssen der Platine zu einem Kurzschluss kommt. Hierzu ist es bevorzugt, wenn die Platine mit beispielsweise einem Kunststoff vergossen ist.

Alternativ oder zusätzlich ist es bevorzugt, wenn eine Abdichtung an einer Achse/ Welle des Innenteils des HF-Aktivierungselements mit einem Elastomer-Dichtring (O-Ring) vorgesehen ist.

Alternativ betrifft die vorliegende Offenbarung ein bipolares HF-Instrument, welches alternativ verwendet werden kann. Das bipolare HF-Instrument ist mit einem Maulteil mit einer ersten Instrumentenbranche und einer zweiten Instrumentenbranche zum Fassen von Gewebe, welche dazu vorgesehen und ausgebildet sind, sich beim Öffnen voneinander weg und beim Schließen aufeinander zu zubewegen, einem Klingenbetätigungselement zum Aktivieren einer mechanischen Klinge in dem Maulteil zum Schneiden von Gewebe, welche dazu vorgesehen und ausgebildet ist, um mittels des Klingenbetätigungselements von einer ersten Position in eine zweite Position bewegbar zu sein, genau zwei HF-Aktivierungsknöpfe zum Aktivieren einer HF-Stromzufuhr zum Versiegeln von Gewebe, und einer, vorzugsweise zuschaltbaren, Einrastvorrichtung vorgesehen, um einen eingerasteten Zustand bei geschlossenen Instrumentenbranchen zu erreichen, wobei das Klingenbetätigungselement als auch das HF-Aktivierungselement derart symmetrisch vorgesehen und ausgebildet sind, um von beiden Seiten des HF-Instruments gleich bedienbar zu sein, wobei jeweils ein HF-Aktivierungselement seitlich auf der ersten und der zweiten Instrumentenbranche angebracht sind.

In anderen Worten kann zur ergonomischen Verbesserung das HF-Aktivierungselement seitlich auf der ersten und der zweiten Instrumentenbranche platziert werden. Damit ist das Instrument ebenfalls vollsymmetrisch. Diese Ausführungsform hat vorzugsweise eine zuschaltbare Einrastvorrichtung, welche mit Hilfe eines proximal angebrachten Drehhebels rotatorisch zuschaltbar ist. Um Sicherzustellen, dass der Anwender bei ausgeschalteter Einrastvorrichtung ausreichend Schließkraft aufbringt, ist eine zusätzliche Kontrolleinrichtung, hier in Form eines dritten Tasters, vorgesehen. Der dritte Taster ist in einer UND-Logik mit den HF-Aktivierungstastern verschaltet und muss permanent betätigt sein, damit die HF-Energiezufuhr aktiviert werden kann. Als weitere Kontrolleinrichtungen sind alternativ oder zusätzlich Sensoren, wie Drucksensoren, Abstandssensoren oder ähnliches vorgesehen. Auf diese Weise werden dem Anwender möglichst viele Anwendungsmöglichkeiten gegeben.

### Kurzbeschreibung der Figuren

Fig. 1 zeigt ein bipolares HF-Instrument in geöffnetem und ausgerasteten Zustand.
Fig. 2 zeigt das bipolare HF-Instrument in geschlossenem und nicht eingerasteten Zustand.
Fig. 3 zeigt das bipolare HF-Instrument in geschlossenem und eingerasteten Zustand.
Fig. 4 zeigt das bipolare HF-Instrument mit offenen Instrumentenbranchen.
Fig. 5 zeigt das bipolare HF-Instrument mit offenen Instrumentenbranchen und der Klinge, wobei ein Teil der ersten Instrumentenbranche und des Maulteils ausgeblendet sind.
Fig. 6 zeigt ein HF-Aktivierungselement gemäß einer ersten Ausführungsform.
Fig. 7 zeigt ein HF-Aktivierungselement in der Draufsicht mit zusätzlich dargestellten elektronischen Tastern und Platine gemäß der ersten Ausführungsform.
Fig. 8 zeigt ein HF-Aktivierungselement gemäß einer zweiten Ausführungsform.
Fig. 9 zeigt ein HF-Aktivierungselement mittels Zahnstange-Zahnrad-Getriebe gemäß einer dritten Ausführungsform.
Fig. 10 zeigt ein HF-Aktivierungselement als Schieber gemäß einer vierten Ausführungsform.
Fign. 11A und 11 B zeigen das HF-Instrument mit einem Hebel als HF-Aktivierungselement.
Fig. 12 zeigt das HF-Instrument aus der Richtung von oben.
Fig. 13 zeigt die Anordnung der HF-Aktivierungstaster und dem Hebel als HF-Aktivierungsknopf.
Fign. 14 und 15 zeigen das bipolare HF-Instrument mit einem HF-Aktivierungselement nach einem zweiten Sicherheitskonzept.
Fign. 16 und 17 zeigen das HF-Instrument mit einem HF-Aktivierungselementverriegelungselement.
Fig. 18, 19 und 20 zeigen das Aktivierungselementverriegelungselement.
Fig. 21 zeigt das Innenteil des HF-Aktivierungselements.
Fig. 22 zeigt die Abstände zwischen den einzelnen Komponenten des HF-Instruments.
Fig. 23 zeigt das HF-Aktivierungselement mit einer Dichtung des HF-Aktivierungselements.
Fig. 24 zeigt einen HF-Aktivierungstaster.
Fig. 25 zeigt das Innenteil eines HF-Aktivierungselements mit Dichtring.
Fig. 26 und 27 zeigen jeweils eine weitere alternative Ausführungsform des HF-Instruments.

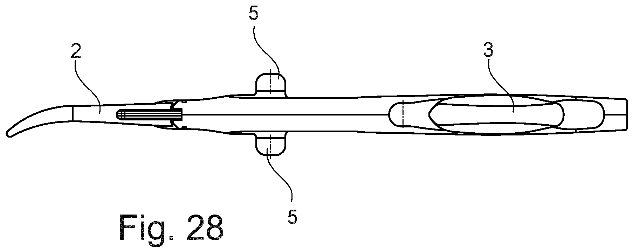

Fig. 28 zeigt das HF-Instrument gemäß Fig. 1 - Fig 5 in einer Draufsicht.

### Beschreibung der Ausführungsformen

Nachstehend werden Ausführungsbeispiele der vorliegenden Offenbarung auf der Basis der zugehörigen Figuren beschrieben.

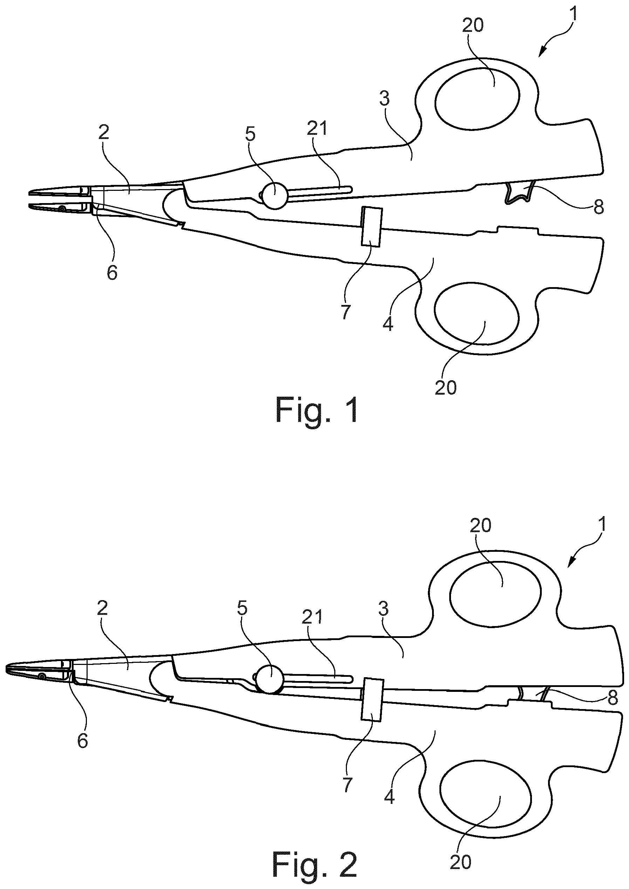

Fig. 1 zeigt ein bipolares HF-Instrument 1 der Scheren- oder Zangenbauart in geöffnetem und ausgerasteten Zustand. Das bipolare HF-Instrument 1 hat ein Maulteil 2, eine erste Instrumentenbranche 3 und eine zweite Instrumentenbranche 4, die über ein Getriebe oder stoffeinstückig mit dem Maulteil gekoppelt sind. Durch das Öffnen und Schließen der ersten Instrumentenbranche 3 und der zweiten Instrumentenbranche 4 verschwenken sich diese um ein nicht weiter in Fig. 1 dargestelltes Scharnier, wodurch sich das Maulteil 2 ebenfalls entsprechend öffnet und schließt. Im geschlossenen Zustand kann das Maulteil 2 Gewebe fassen! greifen/ halten und mittels HF-Energiezufuhr an nicht weiter dargestellte Elektroden im Maulteil 2 versiegelt werden. Dies HF-Technik ist aus dem Stand der Technik beispielsweise gemäß der vorliegenden Anmelderin hinlänglich bekannt.

Die erste Instrumentenbranche 3 und die zweite Instrumentenbranche 4 haben ferner jeweils eine ringförmige Instrumentenbetätigungsöse 20 in der proximalen Hälfte der Instrumentenbranchen 3 und 4, in welche Finger einer Betätigungshand einführbar sind. Andere Instrumentenbetätigungsformen sind natürlich ebenfalls denkbar wie z.B. offene Bügel oder Durchgriffsschlitze in den Instrumentenbranchen 3, 4.

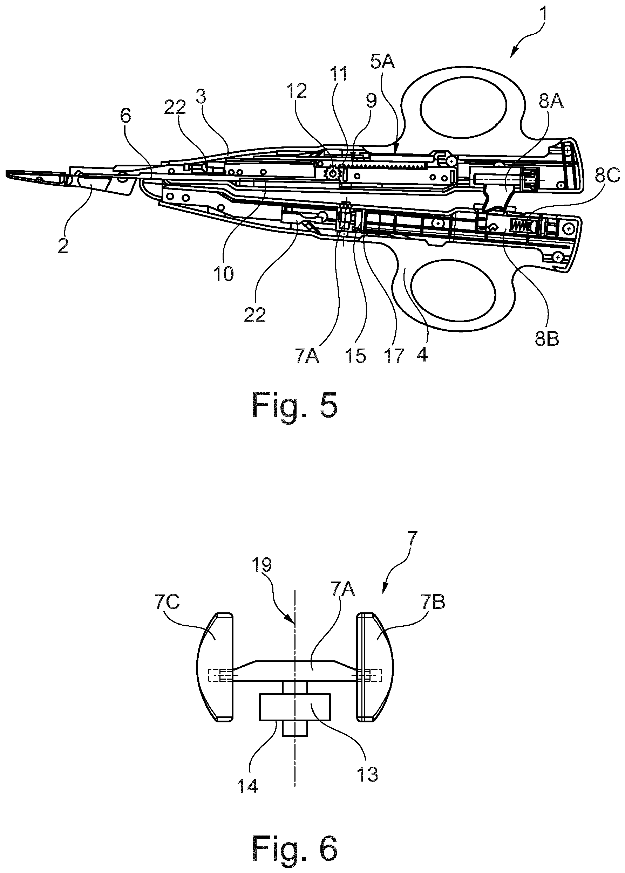

In dem Instrument 1 ist insbesondere im Bereich des Maulteils 2 eine mechanische Klinge 6 vorgesehen, welche mittels eines Klingenbetätigungselements/Tasters/Knopfs 5 vorzugsweise in Instrumentenlängsrichtung bewegbar ist. Die mechanische Klinge 6 ist dafür vorgesehen, das im Maulteil 2 gefasste Gewebe zu schneiden. Die Anordnung der mechanischen Klinge 6 wird in Fig. 5 näher gezeigt.

Das Klingenbetätigungselement 5 ist vorliegend in der ersten Instrumentenbranche 3 angeordnet. Das Klingenbetätigungselement 5 ist derart ausgebildet, linear von seiner distalen Ruheposition nach proximal (d.h. in Richtung weg vom Maulteil 2 / weg vom Patienten) bewegt zu werden. Gemäß Fig. 1 ist das Klingenbetätigungselement 5 innerhalb eines Schlitzes 21 längsverschiebbar gelagert, der in einer Instrumentenbranche 3 ausgeformt ist. Das Klingenbetätigungselement 5 ist dabei zwischen dem Maulteil 2 und der ringförmigen Instrumentenbetätigungsöse 20 der ersten Instrumentenbranche 3 an der zur zweiten Instrumentenbranche 4 angrenzenden Seite angeordnet. Das Klingenbetätigungselement 5 ist vorzugsweise gemäß Fig. 1 als seitlich von der Instrumentenbranche 3 vorragender Stift ausgebildet.

Das Maulteil 2 des bipolaren HF-Instruments 1 weist ferner HF-Litzen (nicht gezeigt) auf, welche nach Betätigung eines HF-Aktivierungselements 7 mit einer HF-Energie versorgt werden und diesen zu den Elektroden im Maulteil 2 weiterleiten. Die HF-Litzen werden an die Flachstecker 22 angeschlossen. Jeweils eine HF-Litze ist mit einem Element des Maulteils 2 verbunden. Die Elemente des Maulteils 2 sind gegeneinander elektrisch isoliert (Isolation nicht gezeigt).

Das HF-Aktivierungselement 7 ist in/an der zweiten Instrumentenbranche 4 an der zur ersten Instrumentenbranche 3 angrenzenden Seite angeordnet. Hierbei ist das HF-Aktivierungselement 7 näher an der ringförmigen Instrumentenbetätigungsöse 20 platziert als das Klingenbetätigungselement 5 und dessen dazugehöriger Schlitz 21 in der ersten Instrumentenbranche 3. Das HF-Aktivierungselement 7 ist vorzugsweise gemäß Fig. 1 als rechteckiger Tipp-Taster ausgebildet.

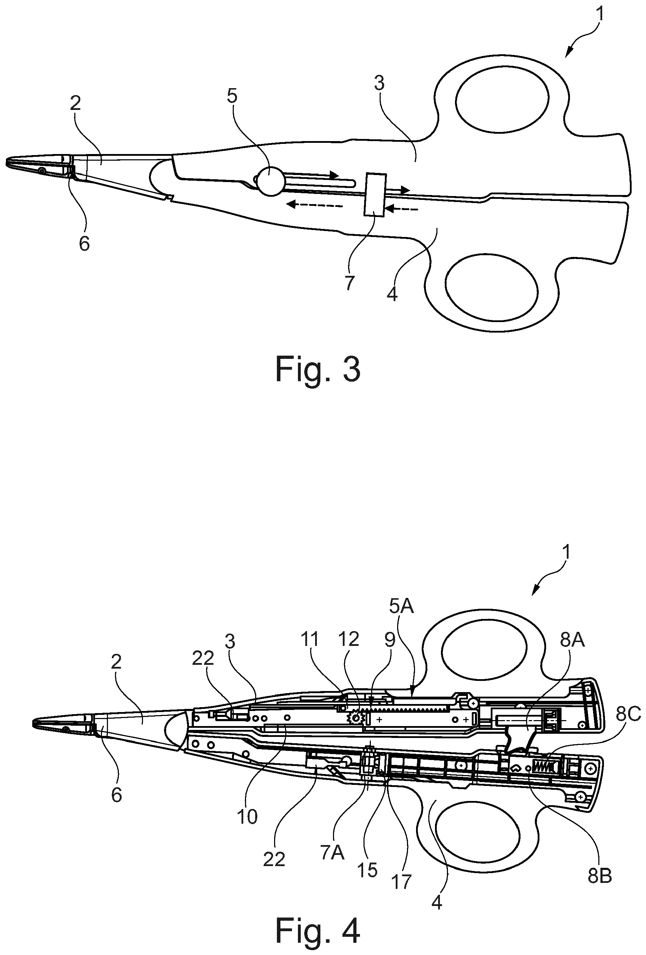

Das bipolare HF-Instrument 1 weist schließlich auf Höhe der ringförmigen Instrumentenbetätigungsösen 20 eine Einrastvorrichtung 8 allgemein bekannten Aufbaus auf. Die Einrastvorrichtung 8 hat einen männlichen Teil 8A und einen weiblichen Teil 8B, welche in Fig. 4 näher dargestellt sind. Die Einrastvorrichtung 8 ist an den Instrumentenbranchen 3 und 4 einander zugewandten Seiten vorgesehen. In Fig. 1 ist die Einrastvorrichtung 8 in einem ausgerasteten Zustand gezeigt.

Fig. 2 zeigt das bipolare HF-Instrument 1 in geschlossenem und (noch) nicht eingerasteten Zustand der Einrastvorrichtung 8. In Fig. 2 ist das HF-Instrument 1 gemäß Fig. 1 gezeigt, wobei die erste Instrumentenbranche 3 und die zweite Instrumentenbranche 4 weiter geschlossen sind. Das Maulteil 2 ist jedoch bereits geschlossen, wobei die Schließkraft entsprechend noch nicht einen bestimmten Wert erreicht hat. In dem in Fig. 2 gezeigten geschlossenen und nicht eingerasteten Zustand ist es je nachdem, welches Sicherheitskonzept verwendet wird, jedoch ggf. bereits möglich, zu schneiden und HF zu aktivieren.

Bei einem weiteren Schließen der Instrumentenbranchen 3 und 4 rastet die Einrastvorrichtung 8 vollständig ein. Fig. 3 zeigt das bipolare HF-Instrument 1 in einem solchen geschlossenen und eingerasteten Zustand. In Fig. 3 ist das HF-Instrument 1 gemäß Fig. 1 und Fig. 2 gezeigt, wobei die erste Instrumentenbranche 3 und die zweite Instrumentenbranche 4 vollständig geschlossen sind. Die Einrastvorrichtung 8 ist eingerastet. In dem eingerasteten Zustand liegen das Klingenbetätigungselement 5 und das HF-Aktivierungselement 7 nahezu in der Mitte zwischen den beiden Instrumentenbranchen 3 und 4 (d.h. im Wesentlichen auf der Symmetrie-Ebene des Instruments 1.

In Fig. 3 ist von dem Klingenbetätigungselement 5 ausgehend ein durchgezogener Pfeil in Richtung proximal dargestellt. Dieser Pfeil stellt die Bewegungsrichtung des Klingenbetätigungselements 5 dar, um die mechanische Klinge 6 zum Schneiden zu bewegen. Ein gestrichelter Pfeil in Richtung distal weisend parallel zu dem durchgezogenen Pfeil zeigt die Richtung des Rückstellwegs des Klingenbetätigungselements 5 an, um die Klinge 6 in Ruhestellung zurückzuführen.

In Fig. 3 ist zudem von dem HF-Aktivierungselement 7 ausgehend ein durchgezogener kurzer Pfeil in Richtung proximal dargestellt. Dieser Pfeil stellt die Bewegungsrichtung des HF-Aktivierungselements 7 dar, um die HF-Energiezufuhr zu aktivieren. Ein gestrichelter Pfeil in Richtung distal weisend parallel zu dem kurzen durchgezogenen Pfeil zeigt die Richtung des Rückstellwegs des HF-Aktivierungselements 7 an.

Fig. 4 zeigt das bipolare HF-Instrument 1 mit offenen Instrumentenbranchen 3 und 4 im Längsschnitt. Die erste Instrumentenbranche 3 und die zweite Instrumentenbranche 4 bestehen aus jeweils zwei Halbgriffschalen, die jeweils einen Hohlraum umschließen, wobei in Fig. 4 das Innenleben der ersten und der zweiten Instrumentenbranche 3 und 4 erkennbar ist.

In Fig. 4 zeigt die Handgriffschale der ersten Instrumentenbranche 3 mit einem erste Zahnstangen-Zahnrad-Getriebe 9 mit einer Input-Zahnstange 10 und einer Output-Zahnstange 11. Die Input-Zahnstange 10 ist mit dem Klingenbetätigungselement 5 (in Fig. 4 nicht gezeigt) fest verbunden. Die Output-Zahnstange 11 ist mit der mechanischen Klinge 6 (in Fig. 4 nicht gezeigt) fest verbunden. Die Input-Zahnstange 10 und die Output-Zahnstange 11 stehen über ein zwischengefügtes Zahnrad 12 derart miteinander in Verbindung, dass bei Betätigung des Klingenbetätigungselements 5 durch einen Anwender in Richtung proximal die mechanische Klinge 6 aus ihrer proximalen Ruheposition nach distal bewegt wird, um das in dem Maulteil 2 gefasste Gewebe zu schneiden. Das Zahnstangen-Zahnrad-Getriebe 9 dient hierbei als Umlenkmechanik in der ersten Instrumentenbranche 3. Für die vorstehende Klingenmechanik ist zudem eine Rückstellfeder 5A derart vorgesehen, um das Klingenbetätigungselement 5 und damit die Klinge 6 automatisch in deren jeweilige Ruheposition zurückzudrängen.

In Fig. 4 zeigt die Handgriffschale der zweiten Instrumentenbranche 4 mit Innenleben des HF-Aktivierungselements 7. Im Einzelnen ist ein Innenteil 7A des HF-Aktivierungselements 7 angedeutet, welches distal zu einem ersten HF-Aktivierungstaster 15 angeordnet ist. Sofern ein zweiter HF-Aktivierungstaster 16 vorgesehen ist, ist dieser seitlich neben dem gezeigten ersten HF-Aktivierungstaster 15 angeordnet und daher in Fig. 4 nicht zu sehen. Der gezeigte HF-Aktivierungstaster 15 ist auf einer Platine 17 angeordnet. Sofern ein erster und ein zweiter HF-Aktivierungstaster 15, 16 vorgesehen sind, sind diese über die Platine 17 miteinander mittels einer ODER-Logik verschalten. Es sind sowohl in der ersten Instrumentenbranche 3 als auch in der zweiten Instrumentenbranche 4 zudem jeweils ein Flachstecker 22 vorgesehen, um die HF-Litzen des Maulteils 2 zu kontaktieren.

Des Weiteren zeigt Fig. 4 die Einrastvorrichtung 8 im Längsschnitt. Die Einrastvorrichtung 8 hat einen männlichen Teil 8A auf Seiten der ersten Instrumentenbranche 3 und einen weiblichen Teil 8B auf Seiten der zweiten Instrumentenbranche 4. Beim Zusammendrücken rasten die beiden Teile 8A und 8B ineinander ein. Durch ein erneutes Zusammendrücken kann der eingerastete Zustand mittels einer eingebauten Rückstellfeder 8C wieder gelöst werden.

Fig. 5 zeigt das bipolare HF-Instrument 1 mit offenen Instrumentenbranchen 3 und 4 und der Klinge 6, wobei ein Teil der ersten Instrumentenbranche 3 und des Maulteils 2 ausgeblendet sind. Fig. 5 zeigt die gleichen Komponenten wie bereits zu Fig. 4 erläutert. Zudem ist die mechanische Klinge 6 gezeigt, welche in dem Maulteil 2 und in der ersten Instrumentenbranche 3 angeordnet und mit der Output-Zahnstange 10 verbunden ist.

Fig. 6 zeigt ein HF-Aktivierungselement 7 gemäß einer ersten nicht zur Erfindung gehörenden Ausführungsform Das HF-Aktivierungselement 7 hat einen Innenteil in Form eines Querriegels 7A, welcher einen ersten Betätigungs-Flügel oder Knopf 7B und einen zweiten Betätigungs-Flügel oder Knopf 7C miteinander verbindet. Gemäß dieser Ausführungsform ist jeweils ein Ende des querriegelförmigen Innenteils 7A in/an dem ersten Betätigungs-Flügel 7B bzw. dem zweiten Betätigungs-Flügel 7C verklebt und/ oder verpresst. Der erste Betätigungs-Flügel 7B und der zweite Betätigungs-Flügel 7C sind außerhalb sowie seitlich, d.h. an den sich gegenüberliegenden bzw. voneinander abgewandten Seiten der zweiten Instrumentenbranche 4 angeordnet. Das Innenteil 7A befindet sich im Innenraum der zweiten Instrumentenbranche 4. In Richtung proximal des HF-Instruments bzw. in Fig. 4 in Richtung nach unten ist ein Kontaktierungselement 13 mit einer Aktivierungsfläche 14 an einem Mittenabschnitt des Innenteils 7A befestigt. Sofern der Anwender das HF-Aktivierungselement 7 betätigt, drückt das mit dem Innenteil 7A verbundene Kontaktierungselement 13 die Aktivierungsfläche 14 auf den HF-Aktivierungstaster 15 (in Fig. 6 nicht gezeigt) und die HF-Energiezufuhr wird aktiviert.

Das Kontaktierungselement 13 ist auf einer Mittelachse 19 des HF-Aktivierungselements 7 d.h. mittig an dem Innenteil 7A T-förmig zu diesem ausgebildet und angebracht, wie dies vorstehend bereits angedeutet ist. Die Aktivierungsfläche 14 ist an der zum HF-Aktivierungstaster 15 zugewandten Seite des Kontaktierungselements 13 angeordnet. Das HF-Aktivierungselement 7 ist längs der Mittelachse 19 wippend in der zweiten Instrumentenbranche 4 gelagert, derart, dass die beiden Betätigungs-Flügel 7B und 7C seitlich an der zweiten Instrumentenbranche vorragen und so von beiden Seiten des Instruments 1 gleich gut erreicht und betätigt werden können.

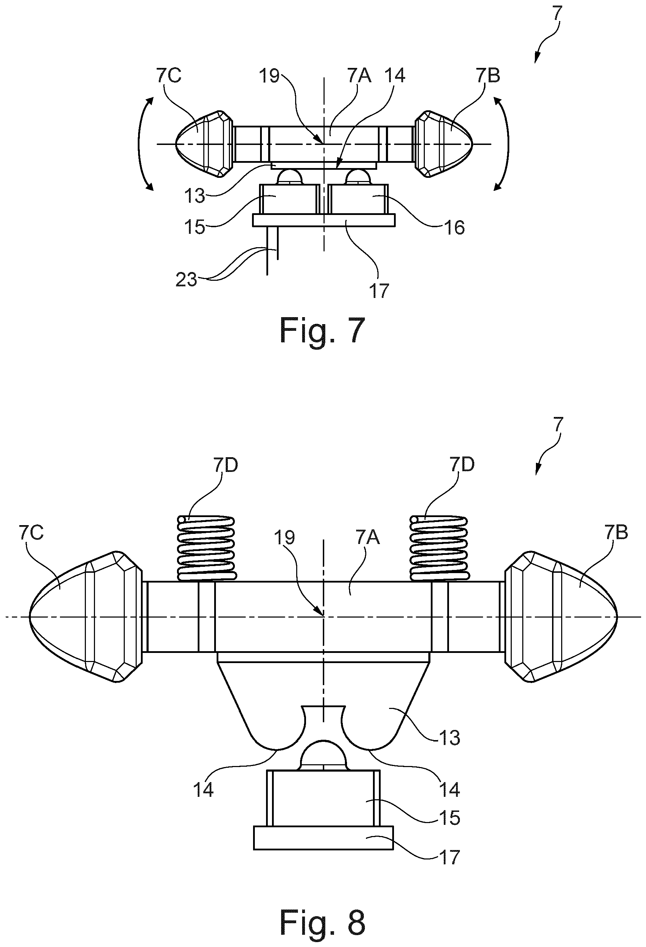

Fig. 7 zeigt das HF-Aktivierungselement 7 gemäß der ersten Ausführungsform. Gemäß Fig. 7 ist ein querriegelförmiges Innenteil 7A des HF-Aktivierungselements 7 mit einem ersten Betätigungs-Flügel 7B und einem zweiten Betätigungs-Flügel 7C gezeigt. Mittig am Innenteil 7A des HF-Aktivierungselements 7 in Richtung hin zu einem ersten und einem zweiten HF-Aktivierungstaster 15 ist ein Kontaktierungselement 13 mit der Aktivierungsfläche 14 an dem Innenteil 7A angebracht oder daran ausgebildet.

In dieser Ausführungsform sind ein erster und ein zweiter HF-Aktivierungstaster 15, 16 vorgesehen, die nebeneinander und in Fig. 7 unterhalb der Aktivierungsfläche 14, d.h. gemäß der Fig. 5 proximal zur Aktivierungsfläche 14 angeordnet sind. Beide HF-Aktivierungstaster 15, 16 sind auf einer (einzigen) Platine 17 angeordnet. An der Platine 17 sind zwei Anschlüsse 23 vorgesehen, welche der Energieversorgung dienen. Durch Verdrehen/ Verschwenken/Wippen des HF-Aktivierungselements 7 in Längsrichtung der Mittelachse 19, unabhängig davon, welchen Betätigungs-Flügel 7B, 7C auf der jeweils gegenüberliegenden Seite des Instruments 1 der Anwender verwendet, drückt die Aktivierungsfläche 14 auf einen der beiden HF-Aktivierungstaster 15, 16, wodurch der HF-Strom auf die Elektroden im Maulteil 2 freigeschaltet wird.

Fig. 8 zeigt ein HF-Aktivierungselement 7 gemäß einer zweiten, erfindungsgemäßen Ausführungsform Die zweite Ausführungsform entspricht nahezu der ersten Ausführungsform, mit dem Unterschied, dass das Kontaktierungselement 13 mit zwei nebeneinander angeordneten nockenförmigen Vorsprüngen ausgeformt ist,, welche den HF-Aktivierungsschalter 7 außenseitig (berührungslos) umgeben, sodass das Kontaktierungselement 13 bzw. dessen Vorsprünge bei Betätigung des HF-Aktivierungselements 7 mit entsprechender Wippenbewegung des querriegelförmigen Innenteils 7A mit dem HF-Aktivierungstaster 15 in Kontakt kommt. Aufgrund des doppel-nockenförmigen Kontaktierungselements 13 ist genau ein elektronischer HF-Aktivierungstaster 15 ausreichend und eine Berührung der Aktivierungsfläche 14 an der zum HF-Aktivierungstaster 15 zugewandten Seite mit dem HF-Aktivierungstaster 15 ist sichergestellt.

Auf der distalen Seite des Innenteils 7A gegenüberliegend zu dem Kontaktierungselement 13 sind (bei allen vorstehend beschriebenen Ausführungsformen des HF-Aktivierungselements 7) zwei in Instrumenten-Querrichtung beabstandete Rückstellfedern 7D vorgesehen, die sich einerseits an der zweiten Instrumentenbranche 4 und andererseits am querriegelförmigen Innenteil 7A abstützen, um unabhängig von welcher Seite des Instruments 1 das HF-Aktivierungselement 7 betätigt wird, diesen in die (kontaktlose) Ausgangsposition zurückzudrängen.

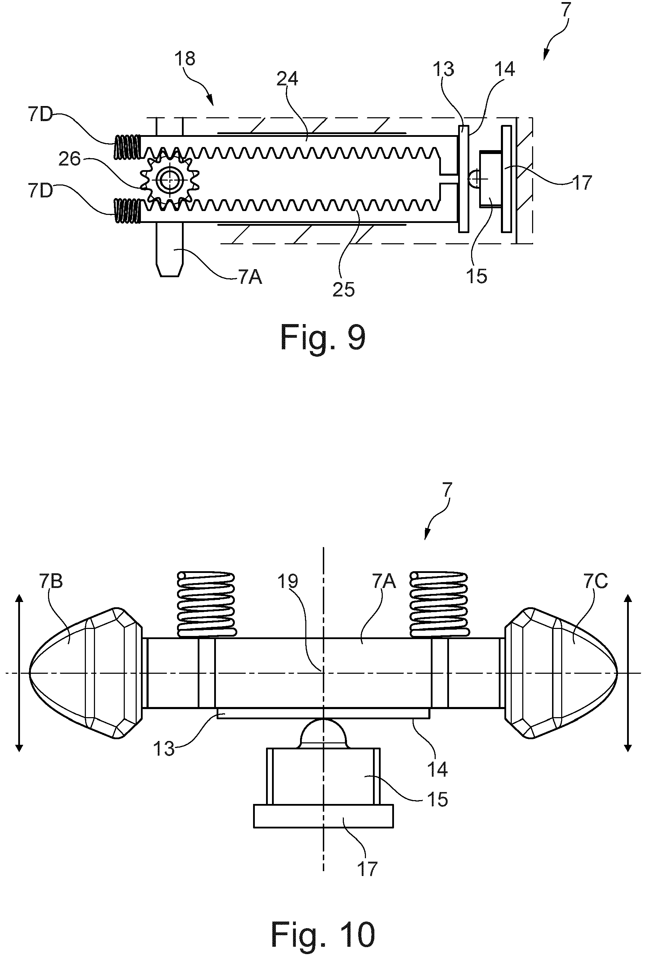

Fig. 9 zeigt ein HF-Aktivierungselement 7 mit einem zweiten Zahnstangen-Zahnrad-Getriebe 18 gemäß einer dritten nicht zur Erfindung gehörenden Ausführungsform. Das zweite Zahnstangen-Zahnrad-Getriebe 18 hat eine erste Zahnstange 24 und eine zweite Zahnstange 25, welche einander gegenüberliegen und an distalen Endabschnitten über ein Zahnrad 26 miteinander in Wirkverbindung stehen. An den distalen Enden, an welchem sich das Zahnrad 26 befindet, ist sowohl an der ersten Zahnstange 24 als auch an der zweiten Zahnstange 25 jeweils eine in Richtung proximal druckwirkende Rückstellfeder 7D angebracht. An den anderen, proximalen Enden der ersten Zahnstange 24 und der zweiten Zahnstange 25 ist ein Kontaktierungselement 13 mit einer Aktivierungsfläche 14 in Richtung hin zu einem proximal zum HF-Aktivierungselement 7 angeordneten HF-Aktivierungstaster 15 vorgesehen. Der HF-Aktivierungstaster 15 ist auf einer Platine 17 angeordnet. Das heißt, das vorstehende Zahnstangen-Zahnrad-Getriebe 18 ist in der zweiten Instrumentenbranche 4 derart vorgesehen, dass der HF-Aktivierungstaster 15 in proximaler Richtung zu dem Zahnstangen-Zahnrad-Getriebe 18 angeordnet ist. Schließlich ist ein Betätigungselement 7A in Form eines Querriegels dargestellt, welches auf einer Seite der zweiten Instrumentenbranche aus diesem vorragt und welches mit dem Zahnrad verbunden ist. Ein weiteres, gleich aufgebautes Betätigungselement (nicht weiter dargestellt) ist ebenfalls mit dem Zahnrad verbunden und ragt auf der gegenüberliegenden Seite der zweiten Instrumentenbranche aus dieser vor.

Wird nunmehr das dargestellte Betätigungselement 7A in Richtung proximal bewegt, verschiebt sich die eine Zahnstange 25 ebenfalls in Richtung proximal wohingegen die andere Zahnstange 24 in Richtung distal bewegt wird. Dadurch erfährt das Kontaktierungselement 13 eine Kippbewegung ähnlich zu den vorstehend beschriebenen Ausführungsformen, wodurch der HF-Kontaktierungstaster 15 betätigt wird. Bei Freigeben des Betätigungselements 7A bewirken die Vorspannfedern 7D ein Rückführen der Zahnstangen 24, 25 in deren Ausgangsposition. Grundsätzlich ist es aber auch möglich, Betätigungselemente mit der Drehachse des Zahnrads 26 zu koppeln, um so die gegenläufigen Längsbewegungen der Zahnstangen 24, 25 über die manuell bewirkte Drehung des Zahnrads 26 auszulösen.

Fig. 10 zeigt ein HF-Aktivierungselement 7 als Schieber gemäß einer vierten nicht zur Erfindung gehörenden Ausführungsform. Die vierte Ausführungsform ist gemäß Fig. 8 ausgebildet, wobei das Kontaktierungselement 13 mit der Aktivierungsfläche 14 flach statt nockenförmig ausgebildet ist. Des Weiteren ist in diesem Fall der querriegelförmige Innenteil 7A nicht wippenförmig wie in der Ausführungsform nach Fig. 8, sondern längsverschiebbar in der zweiten Instrumentenbranche 4 gelagert.

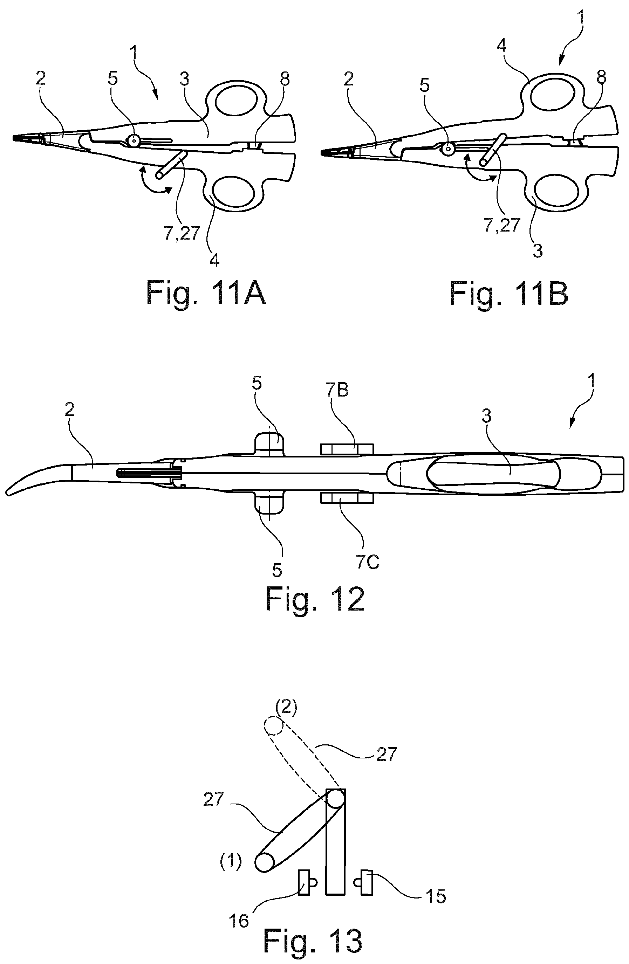

Fign. 11A und 11B und 12 zeigen das HF-Instrument 1 mit einem Hebel 27 als HF-Aktivierungselement 7 jeweils auf den gegenüberliegenden Seiten des Instruments. Diese sechste nicht zur Erfindung gehörende Ausführungsform des HF-Aktivierungselements 7 zeigt gemäß der Fig 11A den einen Hebel 27, welcher seitlich von der zweiten Instrumentenbranche 4 parallel zu dieser schwenkgeführt ist. Hierbei ist in Fig. 11A das HF-Instrument 1 in Übereinstimmung zu den vorstehenden Figuren ausgerichtet. In Fig. 11B ist hingegen die Ausrichtung des HF-Instruments 1 umgedreht, das heißt, die erste Instrumentenbranche 3 ist in Richtung nach unten und die zweite Instrumentenbranche 4 ist in Richtung nach oben abgebildet, sodass beide Hebel 27 auf den gegenüberliegenden Seiten des Instruments dargestellt werden können. Aufgrund der nahezu mittigen Anordnung des HF-Aktivierungselements 7 an der der ersten Instrumentenbranche 3 nahegelegenen Seite der zweiten Instrumentenbranche 4, ist die Bedienung des Hebels 27 unabhängig von der Ausrichtung des HF-Instruments 1 gleich.

Fig. 13 zeigt die Anordnung zweier HF-Aktivierungstaster 15 und 16 und der Hebel 27 als HF-Aktivierungselement 7 gemäß der sechsten Ausführungsform in Rahmen einer Prinzipdarstellung. Gemäß Fig. 13 sind die Hebel 27 mit einem Kontaktstab drehfest verbunden, der sich längs der zweiten Instrumentenbranche 4 vorzugsweise in Richtung proximal erstreckt. Des Weiteren sind ein ersten HF-Aktivierungstaster 15 und ein zweiter HF-Aktivierungstaster 16 vorgesehen, die derart gegenüberliegend zueinander angeordnet sind, um den Kontaktstab zwischen sich zunächst kontaktlos aufzunehmen. Wenn nunmehr der eine, in Volllinie dargestellte Hebel 27 sich in einer ersten Position (1) befindet, in welcher der Kontaktstab keinen Kontakt mit den HF-Aktivierungstastern 15 und 16 hat und aus dieser Position heraus gegen den Uhrzeigersinn betätigt wird, dann wird der erste Aktvierungstaster 15 vom Kontaktstab gedrückt. Für den Fall, dass das Instrument 1 gedreht wird, wie dies in den Fign. 11A und 11B dargestellt ist, kommt der in Strichlinien dargestellte Hebel 27 in seiner zweiten Position (2) in die gleiche räumliche Lage, wie der per Volllinie dargestellte Hebel 27 in Fig. 13, wobei in diesem Fall die beiden HF-Aktivierungstaster 15 und 16 folgerichtig oberhalb des Hebels 27 zu liegen kommen. Wird nunmehr dieser per Strichlinie dargestellte Hebel 27 entgegen dem Uhrzeigersinn betätigt, kommt der Kontaktstab mit zweiten zweite HF-Aktivierungstaster 16 in Kontakt und dieser wird demzufolge gedrückt.

Auch ist es möglich, dass die beiden Hebel 27 ihre jeweils dargestellten Positionen tauschen. In diesem Fall ist eine Umschaltbarkeit von Position (1) zu Position (2) vorzugsweise durch eine Schnappmechanik zwischen Hebel 27 und Kontaktstab realisiert, damit der Hebel spürbar in den definierten Positionen einrastet (nicht gezeigt).

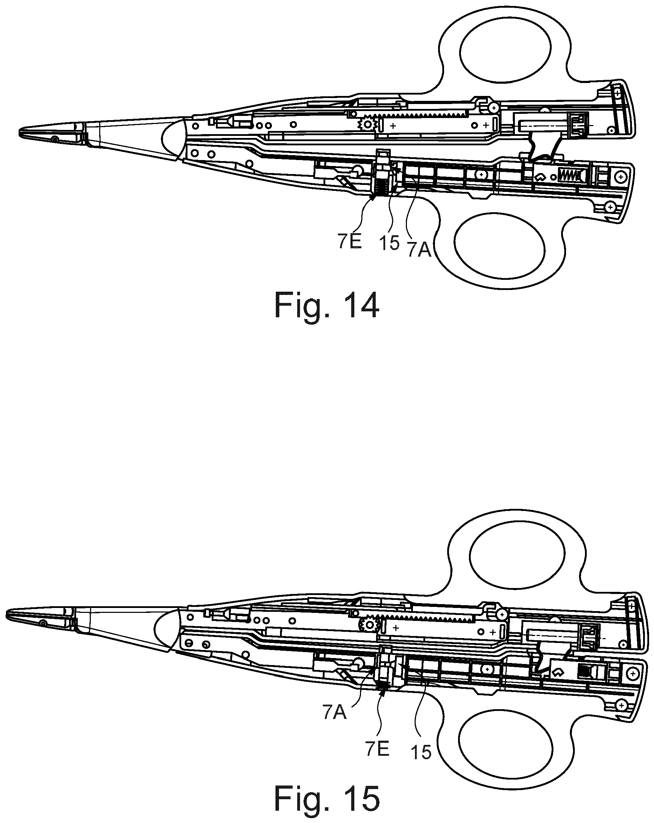

Fign. 14 und 15 zeigen das bipolare HF-Instrument 1 mit einem HF-Aktivierungselement 7 nach einem definierten Sicherheitskonzept. Das HF-Instrument 1 ist in Fig. 14 geschlossen und nicht eingerastet. In Fig. 15 ist das HF-Instrument 1 geschlossen und eingerastet.

Das Innenteil 7A des HF-Aktivierungselements 7 ist mit einem Federelement 7E, vorzugsweise einer Druckfeder, unterbaut. Das Federelement 7E ist derart angeordnet, um zu bewirken, dass das gesamte HF-Aktivierungselement 7 ein Stück weit in Axialrichtung seiner Dreh-/Wippachse 19 aus der Instrumentenbranche 4 herausragt bzw. in Axialrichtung seiner Dreh-/Wippachse 19 verschoben wird. Dies hat gemäß Fig. 14 zur Folge, dass die Aktivierungsfläche 14, die auf den ersten HF-Aktivierungstaster 15 drücken soll, nichtmehr mit dem HF-Aktivierungstaster 15 in Wirkeingriff gebracht werden kann. Beim Betätigen des HF-Aktivierungselements 7 in dieser verschobenen Position kann also keine HF-Energie aktiviert werden.

Sobald das HF-Instrument 1 in einem geschlossenen und eingerasteten Zustand ist, wird durch die erste Instrumentenbranche 3 das HF-Aktivierungselement 7 wieder entgegen der Vorspannkraft des Federelements 7E zurückgedrückt, sodass die Aktivierungsfläche 14 in Eingriff mit dem ersten und/oder zweiten HF-Aktivierungstaster 15 und/oder 16 kommen kann. Dieses Sicherungs- oder Sicherheitskonzept sieht also grundsätzlich ein federkraft-bewirktes Verschieben des HF-Aktivierungselements 7 in eine funktionslose Position vor, die erst mit Erreichen einer vollständigen Schließposition der beiden Instrumentenbranchen 3, 4 wieder aufgehoben wird, indem die andere, erste Instrumentenbranche 3 das HF-Aktivierungselement 7 in seine funktionsbefähigte Position zurückdrückt.

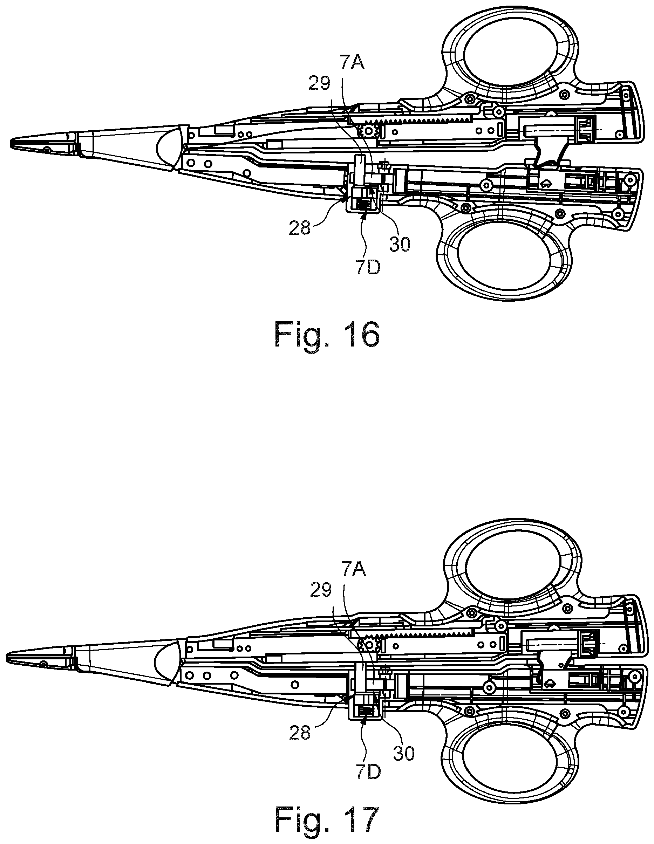

Fign. 16 und 17 zeigen das HF-Instrument 1 mit einem HF-Aktivierungselementverriegelungselement 28 als ein anderes Sicherungs- oder Sicherheitskonzept. Hierbei ist ein HF-Aktivierungselementverriegelungselement 28 in das HF-Instrument 1 eingebaut/ integriert. Demzufolge wird dem Anwender die Betätigung in einem geschlossenen und eingerasteten Zustand ermöglicht, sofern er das HF-Aktivierungselementverriegelungselement 28 entsprechend auslöst.

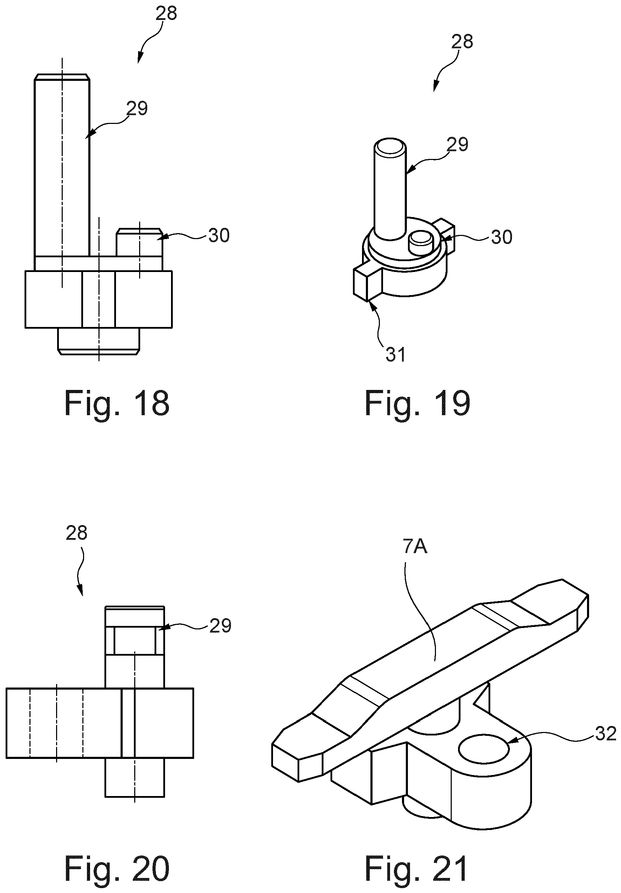

Das Aktivierungselementverriegelungselement 28 gemäß Fign. 18 und 19 hat einen Stößel 29, der aus der Handgriffschale der zweiten Instrumentenbranche 4 herausragt und mit der gegenüberliegenden Handgriffschale der ersten Instrumentenbranche 3 in Kontakt gebracht werden kann, wenn das HF-Instrument 1 geschlossen wird. Neben dem Stößel 29 befindet sich ein Stift 30. Der Stift 30 steht in Interaktion mit einer Bohrung 32 gemäß Fig. 21 im Innenteil 7A des HF-Aktivierungselements 7. In einem offenen Zustand des HF-Instruments 1 gemäß Fig. 16 befindet sich der Stift 30 in der Bohrung 32 und ein Verdrehen/ Wippen/ Betätigen des HF-Aktivierungselements 7 ist nicht möglich. In einem geschlossenen Zustand des HF-Instruments 1 gemäß Fig. 17 wird durch den Stößel 29 der Stift 30 aus der Bohrung 32 geschoben und das Verdrehen/ Wippen/ Betätigen des HF-Aktivierungselements 7 ist möglich.

Um ein Verdrehen des Verriegelungselements 28 zu verhindern, sind auf zwei sich diametral gegenüberliegenden Seiten des Verriegelungselements 28 Führungselemente 31 in Form von Gleitlaschen gemäß Fig. 19 ausgebildet, die in entsprechenden Führungsnuten in der zweiten Instrumentenbranche 4 gleitgelagert sind. Auf diese Weise ist gewährleistet, dass bei einem Öffnen des Instruments der Stift 30 in die Bohrung 32 einfahren kann.

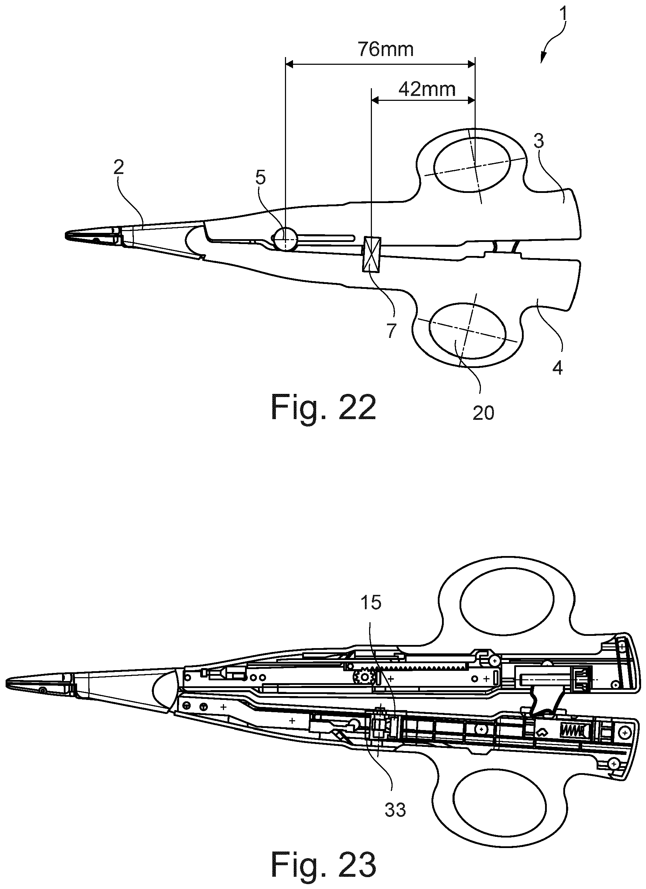

Fig. 22 zeigt die Abstände zwischen den einzelnen Komponenten des HF-Instruments 1. Hierbei sind, um eine Verwechselung des HF-Aktivierungselements 7 und des Klingenbetätigungselements 5 bei der Anwendung zu vermeiden, das HF-Aktivierungselement 7 und das Klingenbetätigungselement 5 in ausreichendem Abstand zueinander platziert. Es ist bevorzugt, wenn der Abstand ausgehend von der Mitte der Instrumentenbetätigungsöse 20 bis zur Mitte des Klingenbetätigungselements 5 vorzugsweise zwischen 56 und 96 mm, weiter bevorzugt zwischen 66 und 86 mm und weiter bevorzugt 76 mm beträgt. Zudem ist es bevorzugt, wenn der Abstand ausgehend von der Mitte der Instrumentenbetätigungsöse 20 bis zur Mitte des HF-Aktivierungselements 7 vorzugsweise zwischen 22 und 62 mm, weiter bevorzugt zwischen 32 und 52 mm und weiter bevorzugt 42 mm beträgt.

Fig. 23 zeigt das HF-Aktivierungselement 7 mit einer Dichtung 33. Fig. 23 zeigt das HF-Instrument 1 gemäß den vorstehenden Beschreibungen, wobei das HF-Aktivierungselement 7 sowie der erste und/ oder zweite HF-Aktivierungstaster 15 und/ oder 16 mit einer TPE-Dichtung 33 abgedichtet ist.

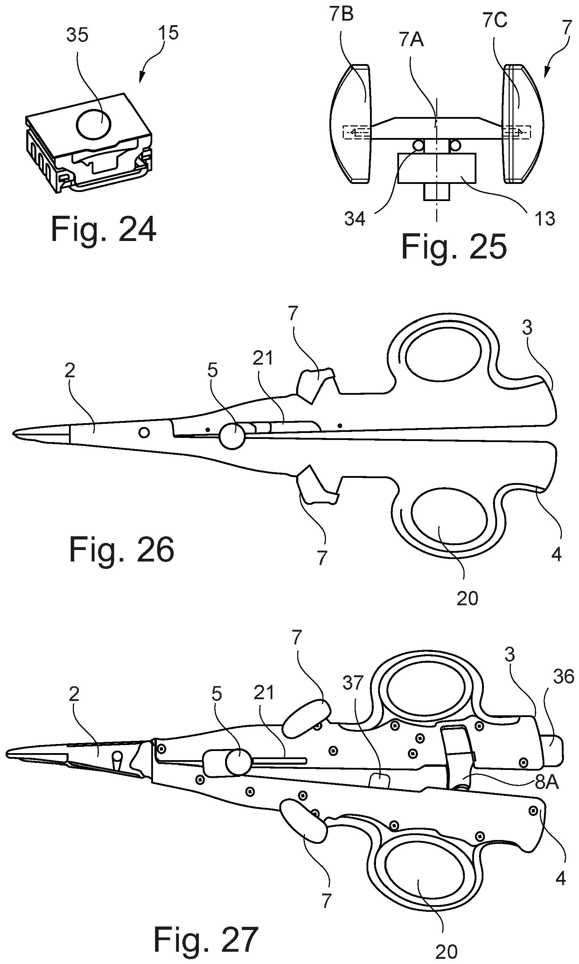

Fig. 24 zeigt einen HF-Aktivierungstaster 15 oder 16, wobei der HF-Aktivierungstaster 15 oder 16 bereits ab Werk mittels einer Ummantelung abgedichtet bzw. gekapselt ist, welche im Kontaktbereich des HF-Aktivierungstasters eine konkave, vorzugsweise halbkugelförmige Ausstülpung aufweist. Bei Berührung der hervorstehenden Halbkugel 35, auf welche die Aktivierungsfläche 14 bei Betätigung des HF-Aktivierungselements 7 drückt, wird die HF-Energie zugeführt.

Fig. 25 zeigt das HF-Aktivierungselement 7 gemäß Fig. 6 mit einem Dichtring 34. Der Dichtring 34 ist um das T-förmige Kontaktierungselement 13 auf der Seite hin zu dem Innenteil 7A des HF-Aktivierungselements 7 gestülpt, sodass der querriegelförmige Innenteil 7A und das Kontaktierungselement 13 samt Kontaktfläche 14 fluiddicht voneinander trennbar sind. Der Dichtring 34 ist vorzugsweise ein Elastomer-Dichtring/ O-Ring.

Fig. 26 und 27 zeigen eine weitere alternative Ausführungsform des HF-Instruments 1. Hierbei ist das HF-Instrument 1 gemäß den vorstehenden Figuren ausgebildet, wobei das HF-Aktivierungselement 7 in doppelter Ausführung vorhanden ist. Die zwei HF-Aktivierungsknöpfe 7 befinden sich in diesem Fall an der Oberseite der jeweiligen Instrumentenbranche 3 und 4, das heißt auf der Ober- und Unterseite des Instruments, wohingegen das Klingenbetätigungselement 5 in Übereinstimmung mit den vorhergehend beschriebenen Ausführungsformen an den linken und rechten Seiten des Instruments verbleiben.

In Fig. 27 ist zudem die Einrastvorrichtung 8, insbesondere der männliche Teil 8A gezeigt, welche mittels eines Drehhebels 36 am proximalen Ende des HF-Instruments 1 zuschaltbar ist. Um sicherzustellen, dass der Anwender bei ausgeschalteter Einrastvorrichtung 8 ausreichend Schließkraft aufbringt, ist eine zusätzliche Kontrolleinrichtung 37 in Form eines Tasters vorgesehen. Die Kontrolleinrichtung 37 ist in einer UND-Logik mit den HF-Aktivierungstaster 15 und/oder 16 verschaltet und muss permanent betätigt sein, um HF-Energie zu aktivieren.

### Bezugszeichen

- 1: Instrument
- 2: Maulteil
- 3: Erste Instrumentenbranche
- 4: Zweite Instrumentenbranche
- 5: Klingenbetätigungselement
- 5A: Rückstellfeder des Klingenbetätigungselements
- 6: Klinge
- 7: HF-Aktivierungselement
- 7A: Innenteil des HF-Aktivierungselements
- 7B: erster Flügel des HF-Aktivierungselements
- 7C: zweiter Flügel des HF-Aktivierungselements
- 7D: Rückstellfeder
- 7E: Federelement
- 8: Einrastvorrichtung
- 8A: männlicher Teil der Einrastvorrichtung
- 8B: weiblicher Teil der Einrastvorrichtung
- 8C: Rückstellfeder der Einrastvorrichtung
- 9: Erstes Zahnstange-Zahnrad-Getriebe
- 10: Input-Zahnstange
- 11: Output-Zahnstange
- 12: Zahnrad
- 13: Kontaktierungselement
- 14: Aktivierungsfläche
- 15: Erster HF-Aktivierungstaster
- 16: Zweiter HF-Aktivierungstaster
- 17: Platine
- 18: Zweites Zahnstange-Zahnrad-Getriebe
- 19: Mittelachse des HF-Aktivierungselementes
- 20: Instrumentenbetätigungsöse
- 21: Schlitz
- 22: Flachstecker
- 23: Anschluss
- 24: Erste Zahnstange
- 25: Zweite Zahnstange
- 26: Zahnrad
- 27: Hebel
- 28: HF-Aktivierungselementverriegelungselement
- 29: Stößel
- 30: Stift
- 31: Führungselement
- 32: Bohrung
- 33: Dichtung
- 34: Dichtring
- 35: Halbkugel
- 36: Drehhebel
- 37: Kontrolleinrichtung

## Patentansprüche

1. Bipolares HF-Instrument (1) mit:
einem Maulteil (2),
einer ersten Instrumentenbranche (3) und einer zweiten Instrumentenbranche (4) zum Fassen von Gewebe, welche dazu vorgesehen und ausgebildet sind, sich beim Öffnen voneinander weg und beim Schließen aufeinander zu zubewegen,
einem vorzugsweise knopf- oder tastenförmigen HF-Aktivierungselement (7) zum Aktivieren einer HF-Stromzufuhr zum Versiegeln von Gewebe,
vorzugsweise einem vorzugsweise hebelförmigen Klingenbetätigungselement (5) zum Bewegen einer mechanischen Klinge (6) in dem Maulteil (2) zum Schneiden von Gewebe,
und vorzugsweise
einer Einrastvorrichtung (8), um einen eingerasteten Zustand bei geschlossenen Instrumentenbranchen (3, 4) zu erreichen,
wobei das HF-Aktivierungselement (7) und vorzugsweise das Klingenbetätigungselement (5) derart vorgesehen und ausgebildet sind, um jeweils von zwei gegenüberliegenden Seiten des HF-Instruments (1) gleichartig bedienbar zu sein,
wobei das HF-Aktivierungselement (7) einen Innenteil (7A) hat, der wippend, schiebbar oder verschwenkbar auf einer Drehachse (19) des HF-Aktivierungselements (7) in der zweiten Instrumentenbranche (4) orthogonal zu der proximal-distal-Richtung gelagert ist, wobei die in Richtung proximal des Instruments (1) weisende Seite des Innenteils (7A) ein Kontaktierungselement (13) mit einer Aktivierungsfläche (14) aufweist, welche zumindest einen ersten HF-Aktivierungstaster (15) bei Betätigung des HF-Aktivierungselements (7) kontaktiert und die HF-Stromzufuhr aktiviert,
wobei der Innenteil (7A) des HF-Aktivierungselements (7) einen ersten Betätigungs-Flügel oder Knopf (7B) und einen zweiten Betätigungs-Flügel oder Knopf (7C) hat, wobei der erste Flügel oder Knopf (7B) seitlich an der zweiten Instrumentenbranche (4) hervorsteht und der zweite Flügel oder Knopf (7C) dem ersten Flügel (7B) gegenüberliegend auf der anderen Seite der zweiten Instrumentenbranche (4) hervorsteht,
**dadurch gekennzeichnet, dass** das Kontaktierungselement (13) mit zwei nebeneinander liegenden Nocken vorgesehen und ausgebildet ist, die sich in Richtung hin zu dem ersten HF-Aktivierungstaster (15) erstrecken und diesen zwischen sich in unbetätigtem Zustand kontaktlos aufnehmen.

2. Bipolares HF-Instrument (1) gemäß Anspruch 1, **dadurch gekennzeichnet, dass** das HF-Aktivierungselement (7) derart ausgebildet und vorgesehen ist, um bei Durchführung einer Versiegelung nach proximal bewegbar insbesondere verschwenkbar oder verschiebbar zu sein.

3. Bipolares HF-Instrument (1) gemäß Anspruch 1 oder 2, **dadurch gekennzeichnet, dass** das HF-Aktivierungselement (7) derart ausgebildet und vorgesehen ist, um nach einer ersten Tipp-Aktivierung die HF-Stromzufuhr zu aktivieren und automatisch, vorzugsweise mittels eines integrierten Algorithmus auszuschalten oder um nach einer zweiten Tipp-Aktivierung des HF-Aktivierungselements (7) die HF-Stromzufuhr manuell zu stoppen.

4. Bipolares HF-Instrument (1) gemäß einem der Ansprüche 1 bis 3, **dadurch gekennzeichnet, dass** der Innenteil (7A) querriegelförmig ist.

5. Bipolare HF-Instrument (1) gemäß einem der Ansprüche 1 bis 4, **dadurch gekennzeichnet, dass** das HF-Aktivierungselement (7) in einem offenen Zustand des HF-Instruments (1) mit einem HF-Aktivierungselementverriegelungselement (28) vorgesehen ist, um eine Betätigung des HF-Aktivierungselements (7) zu verriegeln.

6. Bipolares HF-Instrument (1) gemäß Anspruch 1, **dadurch gekennzeichnet, dass** ein zweiter HF-Aktivierungstaster (16) neben dem ersten HF-Aktivierungstaster (15) parallel zu dem Innenteil (7A) des HF-Aktivierungselements (7) vorgesehen und angeordnet ist.

7. Bipolares HF-Instrument (1) gemäß einem der Ansprüche 1 bis 6, **dadurch gekennzeichnet, dass** der erste HF-Aktivierungstaster (15) und/oder der zweite HF-Aktivierungstaster (16) in proximaler Richtung weg von dem Innenteil (7A) des HF-Aktivierungselements (7) diesem jedoch zugewandt auf einer Platine (17) in der zweiten Instrumentenbranche (4) angeordnet ist/ sind.

8. Bipolares HF-Instrument (1) gemäß Anspruch 1, **dadurch gekennzeichnet, dass** in der ersten Instrumentenbranche (3) ein erstes Zahnstangen-Zahnrad-Getriebe (9) vorgesehen und angeordnet ist, bei welchem eine Input-Zahnstange (10) mit dem Klingenbetätigungselement (5) und eine Output-Zahnstange (11) mit der mechanischen Klinge (6) verbunden ist, wobei das Klingenbetätigungselement (5) für ein Betätigen der Klinge (6) linear von einer distalen Ruheposition nach proximal bewegbar ist.

9. Bipolares HF-Instrument (1) gemäß Anspruch 8, **dadurch gekennzeichnet, dass** die Input-Zahnstange (10) und das damit verbundene Klingenbetätigungselement (5) annähernd auf der Symmetrie-Ebene zwischen der ersten Instrumentenbranche (3) und der zweiten Instrumentenbranche (4) angeordnet sind.

## Claims

1. A bipolar HF instrument (1) comprising:
a jaw part (2),
a first instrument branch (3) and a second instrument branch (4) for grasping tissue, which are provided and configured to move away from each other when opening and toward each other when closing,
a preferably knob-shaped or button-shaped HF activation element (7) for activating an HF power supply for sealing tissue,
preferably a preferably lever-shaped blade actuating element (5) for moving a mechanical blade (6) in the jaw part (2) for cutting tissue,
and preferably
a latching device (8) for achieving a latched-in state with the instrument branches (3, 4) closed,
wherein
the HF activation element (7) and preferably the blade actuating element (5) are provided and configured so as to be operable in the same way from two opposite sides of the HF instrument (1),
wherein the HF activation element (7) has an inner part (7A) which is mounted in a rocking, sliding or pivotable manner on an axis of rotation (19) of the HF activation element (7) in the second instrument branch (4) orthogonal to the proximal-distal direction, wherein the side of the inner part (7A) pointing in the direction proximal to the instrument (1) has a contact element (13) with an activation surface (14) which contacts at least a first HF activation button (15) during actuation of the HF activation element (7) and activates the HF power supply,
wherein the inner part (7A) of the HF activation element (7) has a first actuating wing or knob (7B) and a second actuating wing or knob (7C), wherein the first wing or knob (7B) protrudes laterally on the second instrument branch (4) and the second wing or knob (7C) protrudes opposite the first wing (7B) on the other side of the second instrument branch (4),
**characterized in that** the contact element (13) is provided and configured with two adjacent cams which extend in the direction of the first HF activation button (15) and receive it between them without contact in the non-actuated state.

2. The bipolar HF instrument (1) according to claim 1, **characterized in that** the HF activation element (7) is configured and provided in such a way as to be proximally movable, in particular pivotable or displaceable, when performing sealing.

3. The bipolar HF instrument (1) according to claim 1 or 2, **characterized in that** the HF activation element (7) is configured and provided in such a way that it activates the HF power supply after a first tap activation and switches it off automatically, preferably via an integrated algorithm, or stops the HF power supply manually after a second tap activation of the HF activation element (7).

4. The bipolar HF instrument (1) according to any of claims 1 to 3, **characterized in that** the inner part (7A) is in the shape of a crossbar.

5. The bipolar HF instrument (1) according to any of claims 1 to 4, **characterized in that** the HF activation element (7) is provided in an open state of the HF instrument (1) with a locking element (28) of the HF activation element to lock an actuation of the HF activation element (7).

6. The bipolar HF instrument (1) according to claim 1, **characterized in that** a second HF activation button (16) is provided and arranged next to the first HF activation button (15) in parallel to the inner part (7A) of the HF activation element (7).

7. The bipolar HF instrument (1) according to any of claims 1 to 6, **characterized in that** the first HF activation button (15) and/or the second HF activation button (16) is/are arranged in a proximal direction away from the inner part (7A) of the HF activation element (7), but facing it on a printed circuit board (17) in the second instrument branch (4).

8. The bipolar HF instrument (1) according to claim 1, **characterized in that** in the first instrument branch (3) a first rack and pinion gear (9) is provided and arranged, in which an input rack (10) is connected to the blade actuating element (5) and an output rack (11) is connected to the mechanical blade (6), wherein the blade actuating element (5) is linearly movable proximally from a distal resting position for operating the blade (6).

9. The bipolar HF instrument (1) according to claim 8, **characterized in that** the input rack (10) and the blade actuating element (5) connected thereto are arranged approximately on the plane of symmetry between the first instrument branch (3) and the second instrument branch (4).

## Revendications

1. Instrument HF bipolaire (1) avec :
une partie mâchoire (2),
une première branche d'instrument (3) et une seconde branche d'instrument (4) destinées à la saisie de tissus, qui sont prévues et configurées pour s'éloigner l'une de l'autre lors de l'ouverture et se déplacer l'une vers l'autre lors de la fermeture,
un élément d'activation HF (7) de préférence en forme de bouton ou de touche destiné à l'activation d'une amenée de courant HF destinée au scellement de tissus, de préférence un élément d'actionnement de lame (5) en forme de levier destiné au déplacement d'une lame mécanique (6) dans la partie mâchoire (2) pour la découpe de tissus, et de préférence un dispositif d'encliquetage (8) pour atteindre un état encliqueté lorsque les branches d'instrument (3, 4) sont fermées,
dans lequel l'élément d'activation HF (7) et de préférence l'élément d'actionnement de lame (5) sont prévus et configurés de telle sorte qu'ils soient utilisables de manière similaire respectivement par deux côtés opposés de l'instrument HF (1),
dans lequel l'élément d'activation HF (7) présente une partie interne (7A) qui est montée de manière basculante, coulissante ou pivotante sur un axe de rotation (19) de l'élément d'activation HF (7) dans la seconde branche d'instrument (4) orthogonalement à la direction proximale-distale, dans lequel le côté de la partie interne (7A) orienté dans la direction proximale de l'instrument (1) présente un élément de mise en contact (13) avec une surface d'activation (14) qui entre en contact avec au moins un premier bouton d'activation HF (15) lors de l'actionnement de l'élément d'activation HF (7) et active l'amenée de courant HF,
dans lequel la partie interne (7A) de l'élément d'activation HF (7) présente une première aile d'actionnement ou un premier bouton d'actionnement (7B) et une seconde aile d'actionnement ou un second bouton d'actionnement (7C), dans lequel la première aile ou le premier bouton (7B) fait saillie latéralement au niveau de la seconde branche d'instrument (4) et la seconde aile ou le second bouton (7C) fait saillie à l'opposé de la première aile (7B) de l'autre côté de la seconde branche d'instrument (4),
**caractérisé en ce que** l'élément de mise en contact (13) est prévu et configuré avec deux cames à côté l'une de l'autre qui s'étendent en direction du premier bouton d'activation HF (15) et reçoivent celui-ci entre eux sans contact à l'état désactivé.

2. Instrument HF bipolaire (1) selon la revendication 1, **caractérisé en ce que** l'élément d'activation HF (7) est configuré et prévu de manière à pouvoir se déplacer de manière proximale, en particulier à pouvoir pivoter ou à pouvoir coulisser lors de l'exécution d'un verrouillage.

3. Instrument HF bipolaire (1) selon la revendication 1 ou 2, **caractérisé en ce que** l'élément d'activation HF (7) est configuré et prévu pour activer l'amenée de courant HF après une première activation de toucher et pour désactiver celle-ci automatiquement, de préférence au moyen d'un algorithme intégré ou pour arrêter manuellement l'amenée de courant HF après une seconde activation de toucher de l'élément d'activation HF (7).

4. Instrument HF bipolaire (1) selon l'une quelconque des revendications 1 à 3, **caractérisé en ce que** la partie interne (7A) est en forme de traverse.

5. Instrument HF bipolaire (1) selon l'une quelconque des revendications 1 à 4, **caractérisé en ce que** l'élément d'activation HF (7) est prévu, dans un état ouvert de l'instrument HF (1), avec un élément de verrouillage d'élément d'activation HF (28) pour verrouiller un actionnement de l'élément d'activation (7).

6. Instrument HF bipolaire (1) selon la revendication 1, **caractérisé en ce qu'**un second bouton d'activation HF (16) à côté du premier bouton d'activation HF (15) est prévu et disposé parallèlement à la partie interne (7A) de l'élément d'activation (7).

7. Instrument HF bipolaire (1) selon l'une quelconque des revendications 1 à 6, **caractérisé en ce que** le premier bouton d'activation HF (15) et/ou le second bouton d'activation HF (16) sont pourtant tournés dans la direction proximale à l'écart de la partie interne (7A) de l'élément d'activation HF (7) et disposés sur une platine (17) dans la seconde branche d'instrument (4).

8. Instrument HF bipolaire (1) selon la revendication 1, **caractérisé en ce qu'**un premier entraînement à crémaillère (9) est prévu et disposé dans la première branche d'instrument (3), dans lequel une crémaillère d'entrée (10) est reliée à l'élément d'actionnement de lame (5) et une crémaillère de sortie (11) est reliée à la lame mécanique (6), dans lequel l'élément d'actionnement de lame (5) peut être déplacé de manière linéaire d'une position de repos distale vers une position proximale pour un actionnement de la lame (6).

9. Instrument HF bipolaire (1) selon la revendication 8, **caractérisé en ce que** la crémaillère d'entrée (10) et l'élément d'actionnement de lame (5) relié à celle-ci sont disposés approximativement sur le plan de symétrie entre la première branche d'instrument (3) et la seconde branche d'instrument (4).
